# EUROPÄISCHE PATENTSCHRIFT

(11) **EP 2 297 177 B1**
(45) Veröffentlichungstag und Bekanntmachung des Hinweises auf die Patenterteilung: **20.06.2012**
(21) Anmeldenummer: 09757194.7
(22) Anmeldetag: 26.05.2009
(51) Int. Cl.: C07J 53/00, A61K 31/58, A61P 5/34, A61P 5/42, A61P 5/28

(54) **C-RING-SUBSTITUIERTE PREGN-4-EN-21,17-CARBOLACTONE, SOWIE DIESE ENTHALTENDE PHARMAZEUTISCHE PRÄPARATE**
C-RING-SUBSTITUTED PREGN-4-ENE-21,17-CARBOLACTONE, AND PHARMACEUTICAL PREPARATIONS COMPRISING THE LATTER
PREGN-4-EN-21, 17 CARBOLACTONES SUBSTITUÉS SUR LE NOYAU C, ET PRÉPARATIONS PHARMACEUTIQUES CONTENANT CES DERNIERS

(30) Priorität: 02.06.2008 DE 102008026793
(43) Veröffentlichungstag der Anmeldung: 23.03.2011
(73) Patentinhaber: Bayer Pharma Aktiengesellschaft, 13353 Berlin (DE)
(72) Erfinder: RING, Sven, 07749 Jena (DE); BOHLMANN, Rolf, 14055 Berlin (DE); KUHNKE, Joachim, 14482 Potsdam (DE); ZORN, Ludwig, 13509 Berlin (DE); BORDEN, Steffen, 13355 Berlin (DE); PRELLE, Katja, 40885 Ratingen (DE)
(86) Internationale Anmeldenummer: PCT/EP2009/003716
(87) Internationale Veröffentlichungsnummer: WO 2009/146811

(56) Entgegenhaltungen:
- EP-A- 1 903 051
- WO-A-2007/025780
- US-A- 3 092 628
- US-A- 3 095 412
- US-A- 3 539 558
- MUHN P ET AL: "DROSPIRENONE: A NOVEL PROGESTOGEN WITH ANTIMINERALOCORTICOID AND ANTIANDROGENIC ACTIVITY PHARMACOLOGICAL CHARACTERIZATION IN ANIMAL MODELS" CONTRACEPTION, GERON-X, INC., LOS ALTOS, CA, US, Bd. 51, Nr. 2, 1. Februar 1995 (1995-02-01), Seiten 99-110, XP000908817 ISSN: 0010-7824
- EDWARD A. BROWN ET AL: "Steroidal Aldosterone Blockers. VII" JOURNAL OF MEDICINAL CHEMISTRY., Bd. 6, 1963, Seiten 732-735, XP002546375 AMERICAN CHEMICAL SOCIETY.
- EDWARD A. BROWN ET AL: "Steroidal Aldosterone Blockers. III." JOURNAL OF ORGANIC CHEMISTRY., Bd. 25, 1960, Seiten 96-99, XP002546376 USAMERICAN CHEMICAL SOCIETY. EASTON.

## Beschreibung

Die vorliegende Erfindung betrifft C-Ring-substituierte Pregn-4-en-21,17-carbolactone der allgemeinen Formel I 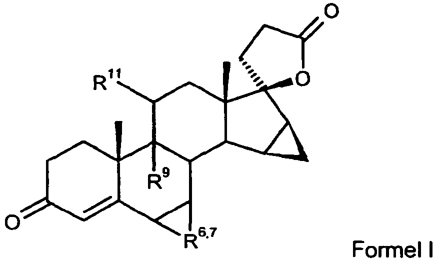 worin
R^{6,7} ein α- oder β-ständiges Methylen und
R⁹ ein Wasserstoffatom und R¹¹ ein Brom-. Chlor- oder Fluoratom oder
R⁹ und R¹¹ gemeinsam eine Bindung
bedeuten.

Das Wasserstoffatom R⁹ befindet sich vorzugsweise in der α-Position.

Das Halogenatom R¹¹ befindet sich vorzugsweise in der β-Stellung.

Als Halogenatom R¹¹ sind ein Fluor- oder Chloratom bevorzugt; ein Fluoratom ist besonders bevorzugt.

Nachstehend genannte Verbindungen sind erfindungsgemäß besonders bevorzugt:
11β-Chlor-6β,7β;15β,16β-dimethylen-3-oxo-17-pregn-4-en-21,17β-carbolacton
6β,7β;15β,16β-Dimethylen-3-oxo-17-pregna-4,9(11)-dien-21,17β-Carbolacton
6β,7β;15β,16β-Dimethylen-11β-fluor-3-oxo-17-pregn-4-en-21,17β-carbolacton
6α,7α;15β,16β-Dimethylen-11β-fluor-3-oxo-17-pregn-4-en-21,17β-carbolacton

Drospirenon (6β,7β-15β,16β-Dimethylen-3-oxo-17-pregn-4-en-21,17β-carbolacton) ist ein neues Gestagen, welches beispielsweise in dem oralen Kontrazeptivum YASMIN^{®} und dem Präparat ANGELIQ^{®} zur Behandlung postmenopausaler Beschwerden enthalten ist. Aufgrund seiner vergleichsweise geringen Affinität zum Gestagenrezeptor und seiner vergleichsweise hohen Ovulationshemmdosis ist

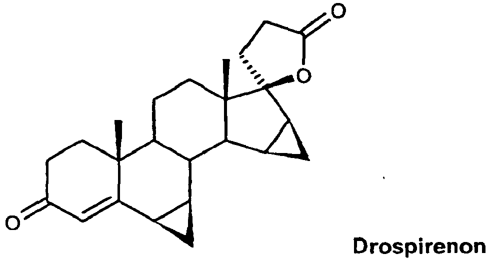

Drospirenon in YASMIN^{®} in der relativ hohen täglichen Dosis von 3 mg enthalten. Drospirenon zeichnet sich dadurch aus, daß es zusätzlich zur gestagenen Wirkung über aldosteronantagonistische (antimineralcorticoide) sowie antiandrogene Wirkung verfügt. Diese beiden Eigenschaften machen das Drospirenon in seinem pharmakologischen Profil dem natürlichen Gestagen Progesteron sehr ähnlich, welches aber anders als das Drospirenon nicht ausreichend oral bioverfügbar ist.

Aufgabe der vorliegenden Erfindung ist es deshalb, Verbindungen zur Verfügung zu stellen, die in vivo über höhere gestagene Potenz als das Drospirenon verfügen sollen. Dies soll sich letztlich in einer geringeren täglichen Dosierung manifestieren und zu einem geringeren Substanzbedarf an aktiver Verbindung führen.

Die durch die vorliegende Erfindung zur Verfügung zu stellenden Verbindungen sollen außerdem in vivo über antimineralcorticoide Wirkung verfügen, die höchstens so hoch wie die des Drospirenons, vorzugsweise aber geringer als diese ist.

Weiterhin sollen die erfindungsgemäßen Verbindungen schwächer antiandrogen wirksam sein als das Drospirenon.

Schließlich sollen die erfindungsgemäßen Verbindungen über hohe metabolische Stabilität verfügen.

Verbindungen, die im Schwangerschaftserhaltungstest an der Ratte eine vielfach höhere Wirksamkeit als das Drospirenon und zum Mineralcorticoidrezeptor aus Rattennierenhomogenat eine dem Drospirenon vergleichbare Aktivität aufweisen, gehen aus der WO2006072467 hervor. Es handelt sich dabei um 18-Methyl-19-nor-17-pregn-4-en-21,17-carbolactone.

Verbindungen, die in vitro über ein weniger dissoziiertes Profil als das Drospirenon hinsichtlich ihrer Bindung an den Progesteron- und Mineralcorticoidrezeptor vefügen, sind in der WO2008000521 beschrieben. Es handelt sich dabei um 18-Methyl-19-nor-androst-4-en-17,17-spiroether.

Ein Verfahren zur Herstellung von 3-Oxo-pregn-4-en-21,17-carbolactonen durch die metallfreie Oxidation von 17-(3-Hydroxypropyl)-3,17-dihydroxyandrostanen ist in der EP 1 746 101 A1 beschrieben. Eine pharmakologische Aktivität generell geht für diese Carbolactone aus der EP 1 746 101 A1 nicht hervor. Als konkrete Verbindung ist lediglich 6β,7β-15β,16β-Dimethylen-3-oxo-17-pregn-4-en-21,17β-carbolacton (Drospirenon) genannt. 11-Halogen- sowie 9,11-Dehydroverbindungen sind spezifisch nicht gezeigt.

Die Aufgabe der vorliegenden Erfindung wird durch die Bereitstellung der hier beschriebenen C-Ring-substituierte Pregn-4-en-21,17-carbolactone der allgemeinen Formel I gelöst. Die Verbindungen der allgemeinen Formel I (und insbesondere die des Beispiels 1 und 2) zeichnen sich durch ein verbessertes Wirkprofil aus..

Die erfindungsgemäßen Verbindungen zeichnen sich durch überraschend starke gestagene Wirksamkeit aus und sind im Schwangerschaftserhaltungs-Test an der Ratte nach subcutaner Applikation stark wirksam.

Die erfindungsgemäßen Verbindungen der allgemeinen Formel I verfügen über stärkere gestagene Wirksamkeit bei gleichzeitiger schwächerer Bindung an den Androgenrezeptor als das Drospirenon.

Es wurde außerdem gefunden, daß die erfindungsgemäßen Verbindungen eine Kaliumretinierende, natriuretische (antimineralcorticoide) Wirkung in adrenalektomierten Ratten zeigen.

Aufgrund ihrer gestagenen Wirksamkeit können die neuen Verbindungen der allgemeinen Formel I allein oder in Kombination mit Estrogen in pharmazeutischen Präparaten zur Kontrazeption verwendet werden.

So können die erfindungsgemäßen Verbindungen der allgemeinen Formel alleine, d. h. ohne Estrogen, zu Herstellung von sogenannten POPs (Progesterone Only Pill) verwendet werden. Solche POPs sind auf Basis anderer Verbindungen mit gestagener Wirksamkeit bereits bekannt, beispielsweise auf Basis des Gestagens Levonorgestrel in Form des Produktes Microlut® (28 tägliche Dosierungseinheiten enthaltend jeweils 30 µg Levonorgestrel).

Wegen ihres günstigen Wirkungsprofils sind die erfindungsgemäßen Verbindungen besonders gut geeignet zur Behandlung prämenstrueller Beschwerden, wie Kopfschmerzen, depressive Verstimmungen, Wasserretention und Mastodynie.

Den erfindungsgemäßen Verbindungen stehen aufgrund ihrer gestagenen Wirksamkeit weitere Verwendungsmöglichkeiten offen, wie diese gemeinhin für Gestagene bekannt sind, so zum Beispiel die Behandlung schwerer Blutungsstörungen, beispielsweise von Menorraghien und Metrorraghien, Behandlung der Corpus Luteum Insuffizienz, d. h. Behandlung eines drohenden Aborts, Behandlung der Pubertas tarda und Behandlung von Zuständen, die eine Gestagensubstitution angezeigt erscheinen lassen.

Zum Gegenstand der vorliegenden Erfindung gehören daher auch pharmazeutische Präparate, die mindestens eine Verbindung der allgemeinen Formel I zusammen mit einem pharmazeutisch verträglichen Träger enthalten.

Erfindungsgemäß sind diejenigen pharmazeutischen Präparate bevorzugt, die 6β,7β;15β,16β-Dimethylen-3-oxo-17-pregna-4,9(11)-dien-21,17β-carbolacton oder 6β,7β;15β,16β-Dimethylen-11β-fluor-3-oxo-17-pregn-4-en-21,17β-carbolacton als Wirkstoff enthalten.

Zum Gegenstand der vorliegenden Erfindung gehören auch pharmazeutische Kombinationspräparate, die neben einer Verbindung der allgemeinen Formel I und dem pharmazeutisch verträglichen Träger ein Estrogen enthalten.

Die Dosierung der erfindungsgemäßen Verbindungen in Kontrazeptionspräparaten soll 0,01 bis 5 mg, vorzugsweise 0,01 bis 2 mg pro Tag betragen.

Die Tagesdosis bei der Behandlung prämenstrueller Beschwerden liegt bei etwa 0.1 bis 20 mg.

Die gestagenen und estrogenen Wirkstoffkomponenten werden in Kontrazeptionspräparaten vorzugsweise zusammen oral appliziert. Die tägliche Dosis wird vorzugsweise einmalig verabfolgt.

Als Estrogene in den erfindungsgemäßen Kombinationspräparaten zur Kontrazeption kommen Estradiol sowie synthetische Estrogene, vorzugsweise Ethinylestradiol, aber auch Mestranol in Betracht.

Außerdem können Ester des Estradiols, hiervon insbesondere das Estradiolvalerat oder auch das -benzoat verwendet werden.

Das Estrogen wird in einer täglichen Menge verabfolgt, die in ihrer estrogenen Wirkung der von 0.01 bis 0.04 mg Ethinylestradiol entspricht.Ethinylestradiol selbst wird in einer täglichen Menge von 0.01 bis 0.04 mg in derartigen Kontrazeptionspräparaten verwendet.

Die neuen Verbindungen der allgemeinen Formel I können auch in pharmazeutischen Präparaten zur Behandlung prä-, peri- und postmenopausaler Beschwerden sowie in Präparaten für die Hormon-Substitutionstherapie (HRT) eingesetzt werden.

Als Estrogene in derartigen Präparaten für die Hormon-Substitutionstherapie kommen in erster Linie natürliche Estrogene zur Anwendung, vor allem das Estradiol oder dessen Ester, beispielsweise Estradiolvalerat oder auch konjugierte Estrogene (CEEs = Conjugated Equine Estrogens), wie sie beispielsweise im Präparat PREMARIN^{®} enthalten sind.

Neuerdings wurde auch beschrieben, Folsäure (WO 99/53910) oder 5-Methyl-6-(S)-tetrahydrofolate, und von diesen insbesondere das Calciumsalz der 5-Methyl-6-(S)-tetrahydrofolsäure (Metafolin®; WO 2006/120035) in Präparate für die Kontrazeption oder die Hormon-Substitutionstherapie einzuarbeiten.

Entsprechende stabile Formulierungen von Tetrahydrofolaten mit einem Gestagen alleine und vor allem mit einem Gestagen und mit einem Estrogen sind in der WO 2008/003432 beschrieben.

Im Falle von Präparaten für die Kontrazeption dient die Folsäure oder die Tetrahydrofolatkomponente der Vorbeugung von Missbildungen des heranreifenden Foetus. Erstrangig geht es dabei um die Vermeidung von NTDs (Neural Tube Defects; Neuralrohrdefekte) im Neugeborenen, einer schwerwiegenden körperlichen Missbildung.

Es liegt im Rahmen der vorliegenden Erfindung, die erfindungsgemäßen, neuen Gestagene analog wie es in den vorstehenden Veröffentlichungen für bereits bekannte Gestagene beschrieben ist, einzusetzen.

Die Formulierung der pharmazeutischen Präparate auf Basis der neuen Verbindungen erfolgt in an sich bekannter Weise, indem man den Wirkstoff, gegebenenfalls in Kombination mit einem Estrogen, mit den in der Galenik gebräuchlichen Trägersubstanzen, Verdünnungsmitteln, gegebenenfalls Geschmackskorrigentien usw., verarbeitet und in die gewünschte Applikationsform überführt.

Sollen die neuen Verbindungen, alleine oder zusammen mit einem Estrogen, gemeinsam mit Folsäure oder mit einem 5-Methyl-6-(S)-tetrahydrofolatverwendet werden, lassen sich entsprechende Formulierungen herstellen, wie es für bereits bekannte Gestagene in den vorstehenden Veröffentlichungen beschrieben ist.

Für die bevorzugte *orale* Applikation kommen insbesondere Tabletten, Dragees, Kapseln, Pillen, Suspensionen oder Lösungen in Frage.

Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Es ist auch möglich, die erfindungsgemäßen Substanzen in ein transdermales System einzuarbeiten und sie damit transdermal zu applizieren.

Ebenso lassen sich die neuen Verbindungen, alleine oder gemeinsam mit einem Estrogen in ein den Wirkstoff oder die Wirkstoffe über einen längeren Zeitraum freisetzendes Verabreichungssystem, beispielsweise ein intrauterines System (IUS), einen Intravaginalring (IVR) oder in ein unter die Haut zu implantierendes System einarbeiten, woraus sie nach dessen Einsetzung in den Uterus, die Vagina oder der Implantation unter die Haut allmählich freigesetzt werden.

### Pharmakologie

### Gestagene Wirkung in ovariektomierten Ratten:

Die gestagene Wirkung wurde bestimmt wie bei Muhn et al. (Muhn, P., Krattenmacher, R., Beier, S., Elger, W., and Schillinger, E. (1995). Drospirenone: a novel progestogen with antimineralocorticoid and antiandrogenic activity. Pharmacological characterization in animal models. Contraception 51, 99-110) beschrieben.

Dabei wird die Fähigkeit der Verbindungen untersucht, in ovariektomierten Tieren, die über keine eigene Progesteronsynthese mehr verfügen, das fehlende Progesteron zu kompensieren und die Schwangerschaft aufrechtzuerhalten ("Schwangerschaftserhaltung").

Weibliche Tiere bei einem Gewicht von 200-230 g wurden verpaart. Die Tiere wurden am Tag 8 post coitum (p.c.) ovariektomiert und mit 5 µg/kg/d Estron behandelt. Die zu testenden Verbindungen wurden in verschiedenen Konzentrationen (3, 10, 30 mg/kg/d) gegeben. Die Behandlung wurde am Tag 8 p.c. begonnen und über 6 Tage fortgesetzt. Auswertung: Einen Tag nach der letzten Behandlung wurden die Tiere autopsiert. Der Schwangerschaftserhaltungsgrad wurde berechnet durch Teilung der Zahl der lebenden Föten durch die Zahl der nachweisbaren Implantationsstellen. Ausschlaggebend für die Einschätzung eines Fötus als lebend war das Vorhandensein eines schlagenden Herzens. Keine erkennbaren Implantationsstellen (ovariektomierte Kontrollen) wurde definiert als 0% Schwangerschaftserhaltung.

Die ED50 (Konzentration, bei der der halbmaximale Effekt auftritt) wurde bestimmt als Maß für die gestagene Potenz.

Es wurde gefunden, dass die erfindungsgemäßen Verbindungen über eine gestagene Potenz verfügen, die bis zu viermal höher liegt als die gestagene Wirkung von Drospirenon.

**Tabelle 1: Gestagene Wirkung der Verbindungen im Schwangerschaftserhaltungstest.**

| | ED₅₀ |
|---|---|
| Drospirenon | 10 mg/kg/d |
| Verbindung des Bsp. 1 | 2.9 mg/kg/d |
| Verbindung des Bsp. 2 | 3.4 mg/kg/d |

### Gestagene Wirkung in ovariektomierten Ratten, Korrelation von Pharmakokinetischen Werten und pharmakodynamischen Effekten

Um den Unterschied in ihrer gestagenen Potenz präziser zu beschreiben, wurden in einem modifizierten Schwangerschaftserhaltungsversuch zusätzlich zu verschiedenen Zeitpunkten Blutproben zur Bestimmung pharmakokinetischer Parameter entnommen. Der Vorbehndlung der Ratten erfolgte dabei wie in vorstehendem Versuch beschrieben. Ein Physiologie-basiertes Pharmakokinetik-Modell wurde entwickelt unter Verwendung der Programme PK-SIM version 4.0.1 (Bayer Technology Services, Leverkusen, Deutschland), GastroPlus version 5.2 (Simulations Plus, Inc., Lancaster, CA, USA) und WinNonlin^{®} Professional (version 5.2, Pharsight Corp., Mountain View, CA, USA).

Die AUC (Fläche unter der Kurve des Zeit-Konzentrationsverlaufes), die während des Experiments erreicht wurde, wurde in Bezug gesetzt zum gemessenen pharmakologischen Effekt, d.h. der erreichten Schwangerschaftserhaltung. Das Modell erlaubte so die Abschätzung von AUC₅₀ Werten.

Ein statistisch signifikanter Unterschied der gestagenen in vivo-Potenzen zwischen Drospirenon und den erfindungsgemäßen Verbindungen wurde gefunden mit mehr als 6-fach niedrigeren AUC₅₀ Werten für die erfindungsgemäßen Verbindungen.. Basierend auf der Beziehung zwischen systemischer Exposition und Wirkungm wurde die AUC in Blut berechnet, die erforderlich ist, um 80% Schwangerschaftserhaltung im Rattenmodell zu erreichen (Tabelle 2).

**Tabelle 2: Erforderliche Exposition zur Erreichung der Schwangerschaftserhaltung (kalkuliert)**

| | AUC (Blut, kalkuliert) in Ratten erforderlich für 80% Schwangerschaftserhaltung |
|---|---|
| Drospirenon | 1346 (µg*h/L) |
| Verbindung des Bsp. 1 | 210 (µg*h/L) |

### Antimineralocorticoide Wirkung der Verbindungen in adrenalektomierten Ratten (Diureseversuche)

Die antimineralocorticoide Wirkung der Verbindungen wurde bestimmt wie bei Losert et al. (Losert, W., Casals-Stenzel, J., and Buse, M. (1985). Progestogens with antimineralocorticoid activity. Arzneimittelforschung 35, 459-471) beschrieben.

Männliche Tiere mit einem Gewicht von 180-200g wurden 5 Tage vor dem Experiment adrenalektomiert und mit Glukokortikoiden substituiert. Am Tag 5 nach der Adrenalektomie wurde ein Diureseexperiment durchgeführt. Den Tieren wurde eine kontinuierliche Infusion isotoner NaCl Lösung plus 5 % Glucose i.v. appliziert. Durch die gleichzeitige Gabe von 1 µg/kg/h d-Aldosteron wurde ein konstanter mineralocorticoider Effekt erzielt, erkennbar an Natriumretention und Kaliurese. Die Testverbindungen wurden in verschiedenen Dosierungen (3, 10 und 30 mg/kg) s.c. appliziert, und die Aufhebung der Aldosteron-induzierten Natriumretention zeigt einen antimineralocorticoiden Effekt an.

### Auswertung:

Die Tiere wurden in Stoffwechselkäfigen gehalten und stündlich Urinfraktionen gesammelt. Die Natrium- und Kaliumionenkonzentration im Urin wurde durch ein flammenphotometrisches Verfahren bestimmt, und daraus der Na/K Quotient berechnet. Die Na/K Quotienten wurden über die Zeit aufgetragen und die Fläche unter der Kurve [AUC] bestimmt.

Die ED₅₀ (Konzentration, bei der der halbmaximale Effekt auftritt) wurde bestimmt als Maß für die antimineralocorticoide Potenz.

Es wurde gefunden, dass die erfindungsgemäßen Verbindungen etwa halb so stark bis etwa gleich stark antimineralcorticoid wirksam sind wie Drospirenon.

**Tabelle 3: Antimineralocorticoide Wirkung der Verbindungen im Diureseversuch.**

| | ED₅₀ |
|---|---|
| Drospirenon | 6.7 mg/kg |
| Verbindung des Bsp. 1 | 5.9 mg/kg/d |
| Verbindung des Bsp. 2 | 12.36 mg/kg/d |

### Antiandrogene Wirkung der Verbindungen in vitro im Antiandrogen-Transaktivierungstest:

Die antiandrogene Wirkung wurde wie bei Schneider et al. beschrieben durchgeführt (Schneider K., Graf E., Irran E., Nicholson G., Stainsby F. M., Goodfellow M., Borden S. A., Keller S., Süssmuth R. D. und Fiedler H. P. (2008) Bendigoles A-C, New Steroids from Gordonia australis Acta 2299, J. Antibiotics (Tokyo) 61 (6), 356 -364).

Zur Kultivierung der für den Test verwendeten Zellen wurde als Kultivierungsmedium RPMI (PAA, #E15-49) mit 10% FCS, 200 mM L-Glutamin, 100 U / 100 µg/ml Penicillin/Streptomycin verwendet. Reporter-Zelllinien (PC3 Zellen stabil transfiziert mit humanem Androgenrezeptor (hAR) sowie einem Reporterkonstrukt, das die Luciferase unter der Kontrolle eines androgen-responsiven Promotors (MMTV) enthält) wurden in einer Dichte von 4 x 10⁴ Zellen pro Vertiefung in weißen, undurchsichtigen Gewebekulturplatten mit jeweils 96 Vertiefungen angezüchtet (Perkin Elmer, #P12-106-017) und in Kultivierungsmedium mit 3 % DCC-FCS (Aktivkohle behandeltes Serum, zur Entfernung im Serum enthaltener störender Komponenten) gehalten. Die zu untersuchenden Verbindungen wurden acht Stunden später zugegeben, und die Zellen wurden mit den Verbindungen 16 Stunden lang inkubiert. Die Versuche wurden dreifach ausgeführt. Am Ende der Inkubation wurde das Effektor enthaltende Medium entfernt und durch Lysis-Puffer ersetzt. Nachdem Luciferase-Assay-Substrat (Promega, #E1501) zugegeben worden war, wurden die Platten mit den 96 Vertiefungen dann in ein Mikroplatten-Luminometer (Pherastar, BMG labtech) eingeführt, und die Lumineszenz wurde gemessen. Die IC₅₀-Werte wurden unter Verwendung einer Software zur Berechnung von Dosis-Wirkungsbeziehungen ausgewertet. Die Efficacy gibt die Prozent des Maximaleffekts im Vergleich zum Maximaleffekt eines Referenzantiandrogens (Hydroxyflutamid) an.

Es wurde gefunden, dass die erfindungsgemäßen Verbindungen *in vitro* schwächer antiandrogen wirksam sind als Drospirenon.

**Tabelle 4: Antiandrogene Wirkung der Verbindungen im Transaktivierungstest.**

| | IC₅₀ [µM] | Efficacy |
|---|---|---|
| Drospirenon | 0.12 | 40.3 |
| Verbindung des Bsp. 1 | 0.24 | 19.3 |
| Verbindung des Bsp. 2 | 0.16 | 31.9 |

### Antiandrogene Wirkung der Verbindungen in orchidektomierten Ratten (Hershberger):

Die antiandrogene Wirkung der Verbindungen wurde bestimmt wie bei Muhn et al. beschrieben (Muhn, P., Krattenmacher, R., Beier, S., Elger, W., and Schillinger, E. (1995).

Drospirenone: a novel progestogen with antimineralocorticoid and antiandrogenic activity. Pharmacological characterization in animal models. Contraception 51, 99-110).

Dabei wird die Eignung der Verbindungen getestet, bei jungen, männlichen, kastrierten und androgensubstituierten Ratten das androgenabhängige Wachstum von Prostata,

Samenblase und M. levator ani zu inhibieren.

Hierfür werden junge Ratten zunächst kastriert. Acht Tage nach der Orchidektomie erhalten die Tiere für sieben Tage 1 mg/kg/Tag Testosteronpropionat (TP) s.c. allein oder in Kombination mit den Testsubstanzen (10 mg/kg/Tag).

Am Tag 15 nach der Orchidektomie werden die Tiere getötet und Prostata, Samenblase und M. levator ani werden präpariert und das relative Feuchtgewicht bestimmt. Das inhibierte androgeninduzierte Wachstum dient als Maß für die antiandrogene Wirkung der Testsubstanz. Der antiandrogene Effekt wurde umgerechnet in Prozent Inhibition, mit vollem Effekt (100% Inhibition) bei einem Prostatagewicht entsprechend der Vehikelkontrolle, und 0% Inhibition bei einem Prostatagewicht entsprechend der TP Behandlung.

**Tabelle 5: Antiandrogene Wirkung der Verbindungen in orchidektomierten Ratten**

| | Antiandrogener Effekt (% Inhibition) |
|---|---|
| Drospirenon | 22.5% |
| Verbindung des Bsp. 1 | < 0% |
| Verbindung des Bsp. 2 | 3.8 % |
| Cyproteronacetat | 73.1 % |

In Übereinstimmung mit ihrem klinischen Profil wurde gefunden, dass Cyproteronacetat einen starken antiandrogenen Effekt zeigt und Drospirenon einen geringeren, aber deutlichen antiandrogenen Effekt. Im Gegensatz dazu wurde gefunden, dass die erfindungsgemäßen Verbindungen keinen antiandrogenen Effekt zeigen.

### Antiandrogene Wirkung der Verbindungen in orchidektomierten Ratten, Untersuchung der Genexpression

Als Modifikation des oben beschriebenen Hershberger Assays wurden zusätzlich Tiere 24 Stunden nach der ersten Behandlung (1 mg/kg TP, 10 mg/kg Testsubstanz) getötet und Prostatagewebe sofort nach der Autopsie schockgefroren und danach zur mRNA Isolation eingesetzt. Durch ein quantitatives PCR-Verfahren (TaqMan) wurde die Induktion (x-facher Induktionsfaktor) von androgen-stimulierten Genen, unter anderen der Steroidbiosynthese (z.B. IDI1, NM_004508.2), als Maß für die androgene Wirkung von TP bzw. deren Inhibition durch die erfindungsgemäßen Verbindungen untersucht. Die Inhibition wurde in % Inhibition umgerechnet mit voller Inhibition (100%) bei einem Induktionsfaktor von 1, und 0% Inhibition bei einem Induktionsfaktor von TP.

**Tabelle 6: Antiandrogene Wirkung der Verbindungen: Genexpressionsveränderung**

| | x-fache Induktionsfaktor von IDI1 mRNA nach TP-Gabe, | Antiandrogener Effekt (% Inhibition) |
|---|---|---|
| Kontrolle (TP) | 7.07 | 0% |
| TP + DRSP | 5.39 | 27 % |
| TP + Bsp. 1 | 7.89 | <0% |
| TP + CPA | 1.53 | 91 % |

| | | |
|---|---|---|
| TP = Testosteronpropionat; DRSP = Drospirenon; CPA = Cyproteronacetat | | |

In Übereinstimmung mit ihrem klinischen Profil wurde gefunden, dass Cyproteronacetat einen starken antiandrogenen Effekt und Drospirenon einen geringeren, aber deutlichen antiandrogenen Effekt zeigt. Im Gegensatz dazu wurde gefunden, dass die erfindungsgemäße Verbindung des Bsp 1 keinen antiandrogenen Effekt zeigt.

Die neuen Verbindungen der allgemeinen Formel I werden erfindungsgemäß wie nachstehend beschrieben hergestellt. Die Syntheseroute für die neuartigen C-Ringsubstituierten Pregn-4-en-21,17-carbolactone gemäß Schema 1 geht zum Beispiel von der bekannten Verbindung 1 [CAS: 95218-07-8, Nickisch et al. J. Med. Chem. 1985, 546-550] aus.

Die Einführung einer Δ^{9,11}-Doppelbindung zum Beispiel über Mesylierung und Abspaltung [Chamberlin et al. J. Org. Chem. 1960, 295] ergibt Verbindung **2** (Beispiel 1).

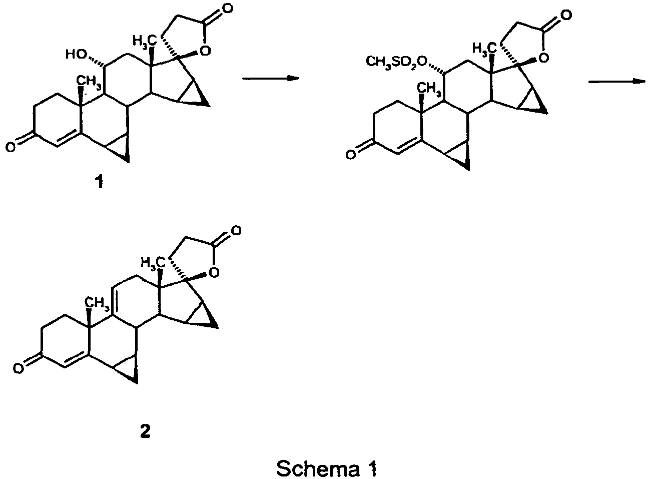

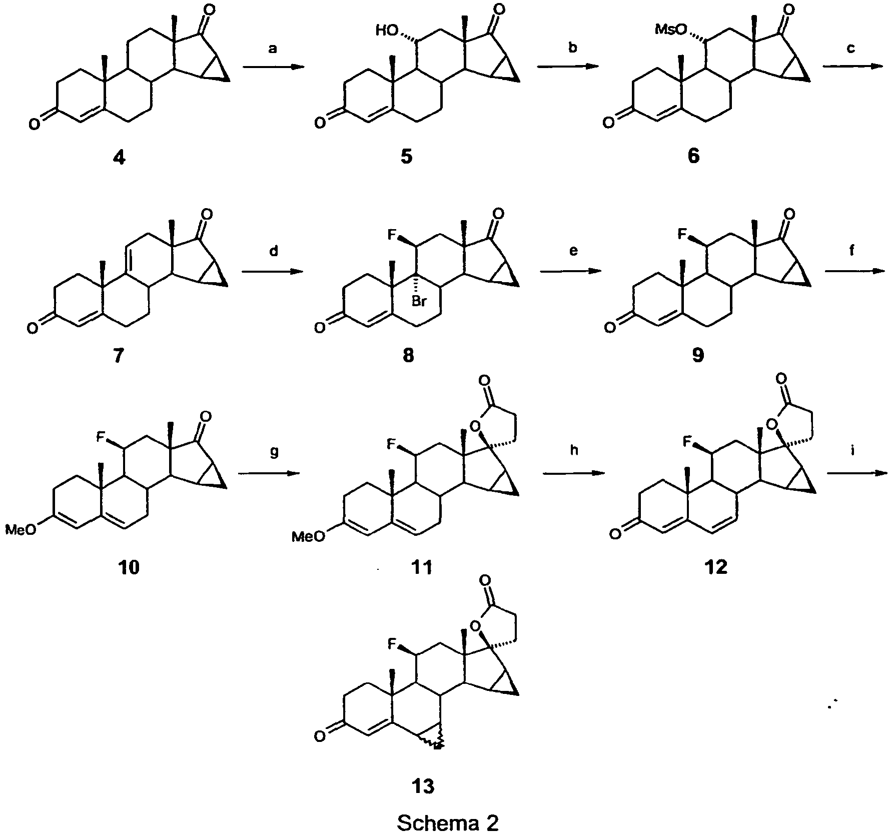

Bedingungen: a) Aspergillus ochraceus; b) CH₃SO₂Cl, Pyridin, DMAP; c) NaOAc, AcOH, Ac₂O, d) Dibromdimethylhydantoin, HF-Pyridin (70%-ig), Dichlormethan; e) Bu₃SnH / AIBN / Benzol; f) 2,2-Dimethoxypropan, Pyridinium-*p*TsOH; g) CH₂=CHCH₂OPO(NMe₂)₂, n-BuLi, THF; h) 1. N-Methylpyrrolidon, NaOAc / H₂O, Dibromdimethylhydantoin, 2. LiBr / Li₂CO₃; i) (CH₃)₃SO-I, DMSO, NaH.

Ein anderes Verfahren vor Herstellung der erfindungsgemäßen Verbindung zeigt das Schema 2.

Aus dem bekannten 15β,16β-Methylen-androst-4-en-3,17-dion [Wiechert et al. Chem. Ber. 106, 1973, 888] (**4**) erhält man (**5**) durch mikrobiologische Hydroxylierung in einem Fermentationsbehälter mit Mikroorganismen, die eine Hydroxylierung des Steroids in der 11-Position, insbesondere der 11α-Position bewirken, z.B. der Spezies Absidia sp., Acremonium sp., Ascochyta sp., Aspergillus sp., Bacillus sp., Beauveria sp., Botryodipoldia sp., Caldariomyces sp., Calonectria sp., Colletotrichum sp., Curvularia sp., Fusarium sp., Gibberella sp., Gloeosporium sp., Glomerella sp., Gnomonia sp., Haplosporella sp., Helicostylum sp., Helminthosporium sp., Metarhizium sp., Mucor sp., Nigrospora sp., Rhizopus sp., Sporotrichum sp., Syncephalastrum sp., und Wojnowicia sp..

Besonders werden Absidia orchidis, Absidia coerulea, Acremonium strictum, Ascochyta clematidina, Aspergillus alliaceus, Aspergillus awamori, Aspergillus fischeri, Aspergillus flavus, Aspergillus malignus, Aspergillus melleus, Aspergillus nidualans, Aspergillus niger, Aspergillus ochraceus, Aspergillus variecolor, Bacillus megaterium, Beauveria bassiana, Beauveria tenella, Botryodiplodia malorum, Caldariomyces fumago, Calonectria decora, Colletotrichum phomoides, Curvularia lunata, Fusarium oxysporium, Fusarium solani, Gibberella zeae, Glomerella cingulata, Gloeosporium fructigenum, Gloeosporium higgensianum, Gloeosporium kaki, Gloeosporium lacticolor, Gloeosporium olivarum, Glomerella fusaroides, Gnomonia cingulata, Haplosporella hesperedica, Helminthosporium sp., Helicostylum piriforme, Metarhizium anisopliae, Mucor plumbeus, Mucor spinosus, Nigrospora sphaerica, Rhizopus arrhizus, Rhizopus cohnii, Rhizopus delemar, Rhizopus japonicus, Rhizopus kazaensis, Rhizopus microsporus, Rhizopus oryzae, Rhizopus shanghaiensis, Rhizopus stolonifer, Rhizopus tritici, Sporotrichum sulfurescens, Syncephalastrum racemosum, Wojnowicia graminis und Wojnowicia hirta verwendet.

Insbesondere werden Absidia orchidis (ATCC 6647), Acremonium strictum (NRRL 5759), Ascochyta clematidina (CBS), Aspergillus alliaceus (ATCC 10060), Aspergillus awamori (CBS), Aspergillus fischeri (ATCC 1020), Aspergillus malignus (IMI 16061), Aspergillus melleus (CBS), Aspergillus nidualans (ATCC 11267), Aspergillus niger (ATCC 9142, ATCC 11394), Aspergillus ochraceus (NRRL405, NRRL 410, CBS 13252, ATCC 46504), Aspergillus variecolor (ATCC 10067), Bacillus megaterium (ATCC 13368), Beauveria bassiana (IFO 5838, ATCC 13144, IFO 4848, CBS 11025, CBS 12736, ATCC 7159), Botryodiplodia malorum (CBS 13450), Caldariomyces fumago (ATCC 16373), Calonectria decora (ATCC 14767), Curvularia lunata (IX 3, NRRL 2380), Fusarium solani (ATCC 12823), Fusarium oxysporum (ATCC 7808), Gibberella zeae (CBS 4474), Glomerella cingulata (ATCC 12097, ATCC 10534, CBS 23849, CBS 23749, ATCC 16646, IFO 6459, IFO 6425, IFO 6470, ATCC 15093, ATCC 10529, IFO 5257, ATCC 56596, ATCC 64682), Glomerella fusaroides (ATCC 9552), Gnomonia cingulata (CBS 15226), Haplosporella hesperedica (CBS 20837), Helicostylum piriforme (ATCC 8992), Helminthosporium sp. (NRRL 4671), Metarhizium anisopliae (IFO 5940), Mucor plumbeus (CBS 29563), Nigrospora sphaerica (ATCC 12772), Rhizopus arrhizus (ATCC 11145), Rhizopus oryzae (ATCC 4858, ATCC 34102, CBS 32947), Rhizopus stolonifer (ATCC 15441), Syncephalastrum racemosum (IFO 4827) und Wojnowicia graminis (CBS 89168) eingesetzt.

Das 11-Hydroxysteroid 5 wird anschliessend z.B. durch Mesylierung und basische Eliminierung der Methansulfonsäure ins Δ⁹⁽¹¹⁾-Derivat **7** überführt. Dieses kann z.B. durch eine Bromfluorierung der Δ⁹⁽¹¹⁾-Doppelbindung nach bekannten Verfahren z.B. mit Olahs Reagenz / N-Bromsuccinimid [Olah et al. Synthesis 1973, 780] in das Dion **8** umgewandelt, und durch reduktive Entbromierung z.B. mit Tributylzinnhydrid ins Fluordion **9** umgewandelt werden. Nach Schutz des 4-En-3-on-Systems (**9**) als Dienolether **10** wird das Spirolacton z.B. nach der Methode von Sturtz [Synthesis 1980, 289] oder alternativ ueber bekannte Verfahren [Bittler Angew. i.e. 21 1982, 696; Laurent J. Steroid Biochem. 19 1983, 771] etabliert. Verbindung **11** kann z.B. durch Dienoletherbromierung analog der Vorschrift von [J.A. Zderic, Humberto Carpio, A. Bowers und Carl Djerassi Steroids 1 1963, 233] und Bromwasserstoffabspaltung durch Erhitzen der 6-Bromverbindung mit basischen Reagenzien, wie z.B. LiBr oder Li₂CO₃ in aprotischen Lösungsmitteln wie z.B. Dimethylformamid oder 1-Methyl-2-pyrrolidon bei Temperaturen von 50-120 °C oder aber indem die 6-Bromverbindungen in einem Lösungsmittel wie Collidin oder Lutidin erhitzt werden ins 4,6-Dien-3-on **12** überführt werden. Verbindung **12** wird dann durch Methenylierung der Δ⁶-Doppelbindung nach bekannten Verfahren z.B. mit Dimethylsulfoxoniummethylid [siehe z.B. DE-A 11 83 500, DE-A 29 22 500, EP-A 0 019 690, US-A 4,291, 029; E. J. Corey und M. Chaykovsky, J.Am. Chem.Soc. 84 1962, 867] in eine Verbindung **13** umgewandelt wobei ein Gemisch der α- und β-Isomeren erhalten wird (das Verhältnis ist abhängig von den verwendeten Substraten, wobei das β-Isomere meist deutlich überwiegt), das z. B. durch Chromatographie in die einzelnen Isomeren getrennt werden kann.

Die Einführung einer 11-Fluorgruppe kann auch nach dem Schema 3 z.B. ausgehend von einem 11-Hydroxy-4-en-on **5** durch Umsetzung mit Nonaflylfluorid und DBU in einem organischen Lösemittel z.B. Tetrahydrofuran [siehe z.B. Bennua-Skalmowski, Tet. Lett. 1995, 2611] unter Bildung einer Mischung des o.g. 11-Fluorsteroids **9** sowie des ebenfalls o.g. Δ⁹⁽¹¹⁾-Derivates 7, die z. B. durch Chromatographie in die einzelnen Verbindungen getrennt werden kann, geschehen und anschließend wie oben beschrieben weiter umgesetzt werden.

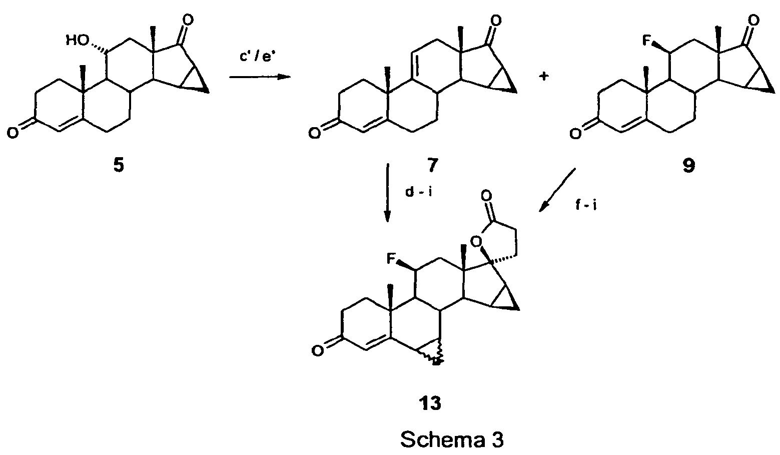

Bedingungen: c' / e') NfF, DBU, THF; d - i) siehe Schema 2.

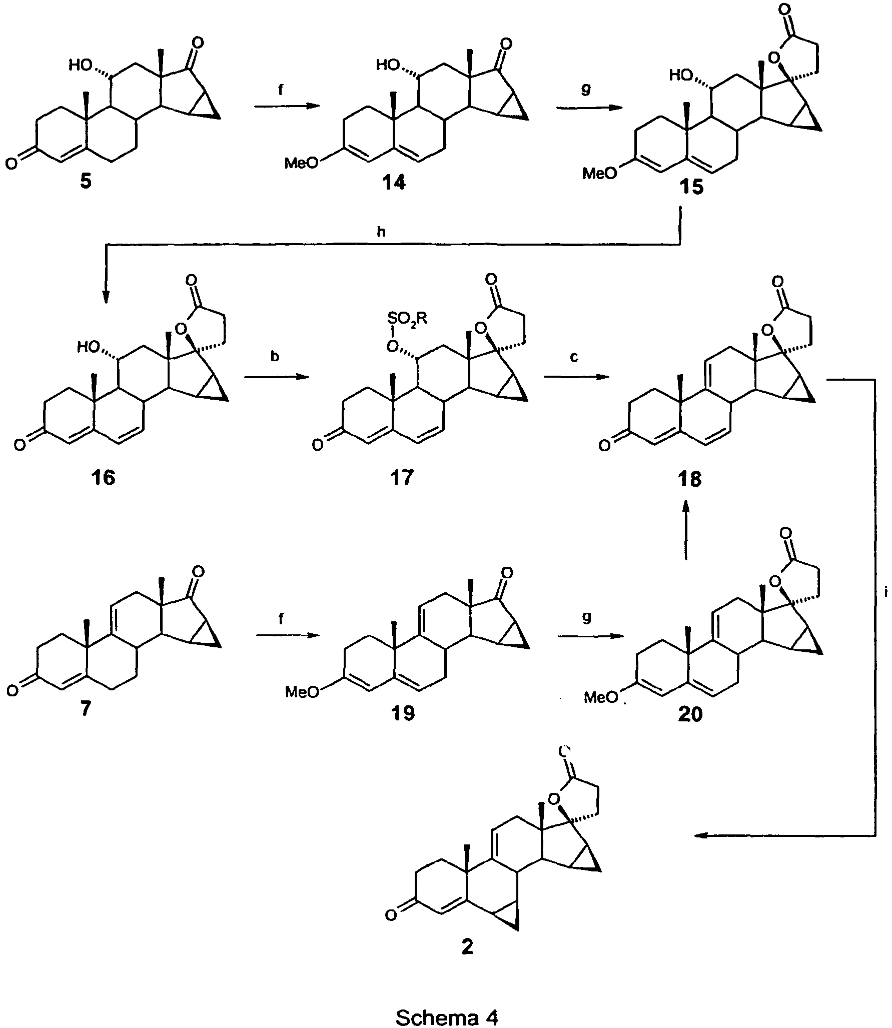

Alle Reaktionsbedingungen siehe Schema 2 (R = Methyl, Tolyl)

Ausgehend von 11-Hydroxysteroid **5** läßt sich in 5 Stufen das 4,6-Dien-3-on **18** nach zuvor beschriebenen Methoden darstellen, aus welchem anschließend durch Methynelierung der 6,7-Doppelbindung (siehe oben) die erfindungsgemäße Verbindung **2** erhalten wird.

Das 4,6-Dien-3-on **18** ist auch ausgehend vom 4-En-3-on **7** in drei Stufen nach zuvor beschriebenen Methoden zugänglich.

Bei den Zwischenverbindungen der Formeln **5, 6, 7, 8, 9, 10, 11, 12, 14, 15, 16, 17, 18, 19 und 20** handelt es sich durchweg um neue Verbindungen. Sie gehören daher alle zum Gegenstand der vorliegenden Erfindung.

Außerdem gehört ihre Verwendung als Ausgangs- bzw. Zwischenverbindungen zur Herstellung der erfindungsgemäßenverbindungen der allgemeinen Formel I zum Gegenstand der vorliegenden Erfindung.

Die nachfolgenden Beispiele dienen der näheren Erläuterung der Erfindung:

### Beispiel 1

### 6β,7β;15β,16β-Dimethylen-3-oxo-17-pregna-4,9(11)-dien-21,17β-carbolacton

### a) 6β,7β;15β,16β-Dimethylen-11α-mesyloxy-3-oxo-17-pregn-4-en-21,17β-carbolacton

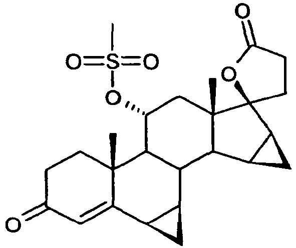

Eine Lösung von 25 g 6β,7β;15β,16β-Dimethylen-11a-hydroxy-3-oxo-17-pregn-4-en-21,17β-carbolacton [CAS: 95218-07-8, Nickisch et al. J. Med. Chem. 1985, 546-550] in 250 ml Pyridin wurde bei 0 °C tropfenweise mit 21.7 ml Mesylchlorid versetzt, 2 Stunden bei 25 °C gerührt. Anschließend verdünnte man mit Ethylacetat, wusch mit Natriumhydrogencarbonatlösung, Wasser und Kochsalzlösung neutral, trocknete über Natriumsulfat, und engte im Vakuum bei 40 °C ein. Man erhielt 30 g reines 6β,7β;15β,16β-Dimethylen-11α-mesyloxy-3-oxo-17-pregn-4-en-21,17β-carbolacton als Feststoff.
¹H-NMR (600 MHz, CDCl₃): δ = 6.01 (s, 1 H), 5.46 (d(br), 1 H), 2.69-2.61 (m, 2H), 2.56-2.46 (m, 3H), 2.39 (m, 1H), 2.27 (d(br), 1H), 2.19-2.13 (m, 2H), 1.89 (m, 1H), 1.81 (dd(br), 1H), 1.72 (m, 1H), 1.67 (m, 1 H), 1.52-1.45 (m, 2H), 1.37 (m, 1 H), 1.31 (m, 1 H), 1.28 (s, 3H), 1.06 (m, 1H), 0.94 (s, 3H), 0.58 (m, 1H).

### b) 6β,7β;15β,16β-Dimethylen-3-oxo-17-pregna-4,9(11)-dien-21,17β-carbolacton

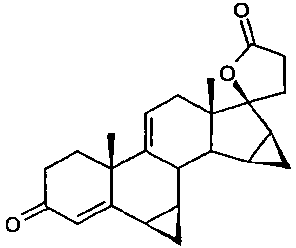

Eine Lösung von 18.5 g 6β,7β;15β,16β-Dimethylen-11α-mesyloxy-3-oxo-17-pregn-4-en-21,17β-carbolacton in 50 ml Essigsäure wurde bei 25 °C mit 0.5 ml Acetanhydrid versetzt, 8 Stunden bei 100 °C Badtemperatur gerührt. Anschließend gab man auf Wasser, extrahierte dreimal mit Ethylacetat, wusch mit Wasser und Kochsalzlösung neutral, trocknete über Natriumsulfat, und engte im Vakuum bei 40 °C ein. Man erhielt 15.2 g rohes 6β,7β-15β,16β-Dimethylen-3-oxo-17-pregna-4,9(11)-dien-21,17β-carbolacton. Nach Chromatrographie an Kieselgel mit Hexan / Ethylacetat erhielt man 7.5 g reines Produkt als Feststoff.
MS (EI): m/z = 364 (M⁺);
¹H-NMR (400 MHz, CDCl3): δ = 6.07 (s, 1H), 4.96 (m, 1H), 2.97 (s, 3H), 2.75-2.51 (m, 3H), 2.47-2.31 (m, 3H), 2.22-1.84 (m, 5H), 1.77-1.41 (m, 8H), 1.32-1.23 (m, 2H),1.03 (s, 3H), 0.84 (m, 1 H).

### Beispiel 2

### 6β,7β;15β,16β-Dimethylen-11β-fluor-3-oxo-17-pregn-4-en-21,17β-carbolacton

### a) 11α-Hydroxy-15β,16β-methylen-androst-4-en-3,17-dion

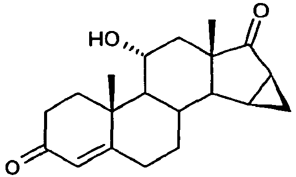

Ein 2-L-Erlenmeyerkolben, der 1 l einer 30 Minuten bei 121 °C im Autoklaven sterilisierten Nährlösung aus 3% Glucose-Monohydrat, 1% Maisquellwasser, 0.2% Natriumnitrat, 0.1% Kaliumdihydrogenphosphat, 0.2% Dikaliumhydrogenphosphat, 0.05% Kaliumchlorid, 0.05% Magnesiumsulfat Heptahydrat und 0.002% Eisen(II)sulfat Heptahydrat (auf pH 6.0 eingestellt) enthielt, wurde mit einer 2 ml DMSO-Eiskultur des Stammes *Aspergillus ochraceus* (NRRL 405) beimpft und 71.5 Stunden bei 27 °C auf einem Rotationsschüttler mit 165 Umdrehungen pro Minute geschüttelt. Mit dieser Vorzucht wurde ein 20-L-Fermenter beimpft, der mit 19 l sterilem Medium der gleichen Endzusammensetzung, wie für die Vorkultur beschrieben, beschickt war. Außerdem wurden vor dem Sterilisieren noch 1.0 ml Siliconöl und 1.0 ml Synperonic zur Schaumbekämpfung zugegeben. Dieser Fermenter wurde 47.5 Stunden bei 0.7 bar Überdruck, einer Temperatur von 28 °C, einer Belüftung von 8 l pro Minute und einer Rührgeschwindigkeit von 350 Umdrehungen pro Minute inkubiert.

Aus diesem 20-L-Fermenter wurden 2.5 l Vorkultur entnommen, um einen 50-L-Fermenter zu beimpfen, der mit 47.5 l sterilem Medium der gleichen Endzusammensetzung, wie für die Vorkultur beschrieben, beschickt war. Vor dem Sterilisieren wurden 2.5 ml Siliconöl und 2.5 ml Synperonic zugegeben. Nach einer Anwachsphase von 10 Stunden bei 0.7 bar Überdruck, einer Temperatur von 28 °C, einer Belüftung von 10 l pro Minute und einer Rührgeschwindigkeit von 350 Umdrehungen pro Minute wurde eine Lösung aus 10.0 g 15β,16β-Methylen-androst-4-en-3,17-dion in 200 ml DMF hinzugegeben. Es wurde weitergerührt und belüftet. Nach 26 Stunden wurde die Kulturbrühe geerntet.

Aus dem 20-L-Fermenter wurden 5.0 l Vorkultur entnommen, um einen 100-L-Fermenter zu beimpfen, der mit 95.0 l sterilem Medium der gleichen Endzusammensetzung, wie für die Vorkultur beschrieben, beschickt war. Vor dem Sterilisieren wurden 5.0 ml Siliconöl und 5.0 ml Synperonic zugegeben. Nach einer Anwachsphase von 10 Stunden bei 0.7 bar Überdruck, einer Temperatur von 28 °C, einer Belüftung von 20 l pro Minute und einer Rührgeschwindigkeit von 350 Umdrehungen pro Minute wurde eine Lösung aus 20.0 g 15β,16β-Methylen-androst-4-en-3,17-dion in 400 ml DMF hinzugegeben. Es wurde weitergerührt und belüftet. Nach 26.25 Stunden wurde die Kulturbrühe geerntet.

Die beiden Kulturbrühen wurden vereinigt und mit 60 l Methyl-iso-butylketon 19.75 Stunden extrahiert. Die vereinigten organischen Phasen wurden bis zur Trockene eingeengt. Der Rückstand wurde mit Hexan gewaschen, um das Siliconöl abzutrennen. Anschließend wurde aus Aceton kristallisiert und 19.2 g (61% d. Th.) 11β-Hydroxy-15β,16β-methylen-androst-4-en-3,17-dion isoliert.

100 mg wurden über eine präparative HPLC gereinigt (250 x 40 mm, Luna C18, 10 µ, 100 A, Wasser-Acetonitril 70 : 30, 100 ml/min).
Fp.: 2251247-249 °C
[α]_{D}= +48.6° (CHCl₃, c = 1.0700)
¹H-NMR (400 MHz, CDCl3): δ = 1.04 (s, 3H), 1.13-1.37 (m, 8H), 1.62 (dt, 1 H), 1.77-1.91 (m, 2H), 1.99 (m, 1H), 2.03-2.21 (m, 4H), 2.31-2.57 (m, 5H), 4.05 (m, 1 H), 5.78 (s, 1H).

### b) 11α-Mesyloxy-15β,16β-methylen-androst-4-en-3,17-dion

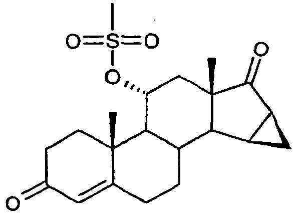

Eine Lösung von 22 g 11α-Hydroxy-15β,16β-methylen-androst-4-en-3,17-dion in 220 ml Pyridin wurde bei 0 °C tropfenweise mit 23 ml Mesylchlorid versetzt, 2 Stunden bei 25 °C gerührt. Anschließend verdünnte man mit Ethylacetat, wusch mit Natriumhydrogencarbonatlösung, Wasser und Kochsalzlösung neutral, trocknete über Natriumsulfat und engte im Vakuum bei 40 °C ein. Man erhielt 24,7 g 11α-Mesyloxy-15β,16β-methylen-androst-4-en-3,17-dion.
¹H-NMR (600 MHz, CDCl3): δ = 5.81(m, 1H), 5.09(m, 1H), 1.39 (s, 3H), 1.21 (m,1H) 1.06 (s, 3H).

### c) 15β,16β-Methylen-androsta-4,9(11)-dien-3,17-dion

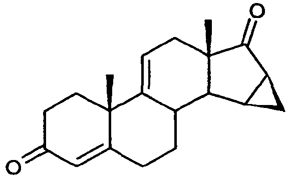

Eine Lösung von 25.6 g 11α-Mesyloxy-15β,16β-methylen-androst-4-en-3,17-dion in 80 ml Essigsäure wurde bei 25 °C mit 0.82 ml Acetanhydrid versetzt und 8 Stunden bei 100 °C Badtemperatur gerührt. Anschließend gab man auf Wasser, extrahierte dreimal mit Ethylacetat, wusch mit Wasser und Kochsalzlösung neutral, trocknete über Natriumsulfat, und engte im Vakuum bei 40 °C ein. Nach Kristallisation aus Ethylacetat erhielt man 16,4 g 15β,16β-Methylen-androsta-4,9(11)-dien-3,17-dion.
¹H-NMR (600 MHz, CDCl3): δ = 5.79(m, 1 H), 5.55(m, 1H), 1.85(m,1H), 1.65(m,1H), 1.37(s, 3H), 1.12-1.33 (2m, 2H), 1.00 (s, 3H).

### d) 9α-Brom-11β-fluor-15β,16β-methylen-androst-4-en-3,17-dion

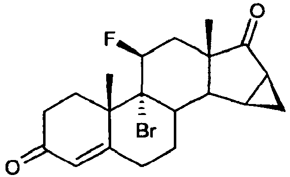

Eine Suspension von 8.76 g Dibromhydantoin in 250 ml Dichlormethan wurde mit 24.5 ml HF / Pyridin 70%ig langsam versetzt. In die erhaltene Lösung wurden 16.3 g 15β,16β-Methylen-androsta-4,9(11)-dien-3,17-dion eintragen und 30 min bei Raumtemperatur gerührt. Anschließend goß man auf ein Gemisch aus 200 ml Ammmoniakwasser (25%) und 300 ml Eis, extrahierte dreimal mit Ethylacetat, wusch mit Wasser und Kochsalzlösung neutral, trocknete über Natriumsulfat und engte im Vakuum bei 40 °C ein. Nach Kristallisation des Rückstandes aus Ethylacetat erhielt man 15,2 g 9α-Brom-11β-fluor-15β,16β-methylen-androst-4-en-3,17-dion.
¹H-NMR (600 MHz, CDCl3): δ = 5.81 (m, 1 H) 5.28(dt, 1 H), 1.695(d, 3H), 1.175 (d, 3H).

### e) 11β-Fluor-15β,16β-methylen-androst-4-en-3,17-dion

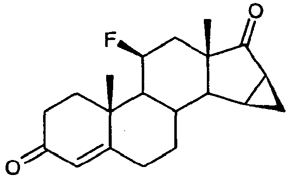

Eine Lösung von 33.5 g 9α-Brom-11β-fluor-15β,16β-methylen-androst-4-en-3,17-dion in 480 ml Benzol wurde mit 42 ml Tributylzinnhydrid und 416 mg Azo-bis-isobutyronitril 30 min bei 80 °C gerührt. Es wurde im Vakuum eingeengt und der Rückstand an Kieselgel 60 chromatografiert. Nach Kristallisation aus Ethylacetat erhielt man 18,7 g 11 β-Fluor-15β,16β-methylen-androst-4-en-3,17-dion.
¹H-NMR (600 MHz, CDCl3): δ = 5.73(m, 1 H), 5.28(dq, 1 H), 1.395(d, 3H), 1.175 (d, 3H).

### f) 11β-Fluor-3-methoxy-15β,16β-methylen-androsta-3,5-dien-17-on

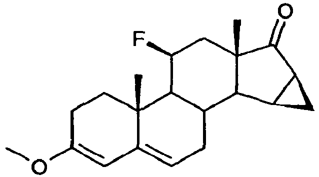

In eine Suspension von 10.79 g 11β-Fluor-15β,16β-methylen-androst-4-en-3,17-dion in 220 ml 2,2-Dimethoxypropan wurden 1.3 g Pyridintosylat eintragen. Dann wured 3 h bei 100°C Badtemperatur gerührt. Nach dem Abkühlen auf Raumtemperatur gab man 2.5 ml Triethylamin zu und engte im Vakuum zur Trockne ein. Der Rückstand wurde mit 30 ml Methanol ausgerührt und abgesaugt. Man erhielt 9,6 g 11β-Fluor-3-methoxy-15β,16β-methylen-androsta-3,5-dien-17-on.
¹H-NMR (600 MHz, CDCl3): δ = 5.27-5.19 (m, 1.5H), 5.14 (m, 1 H), 5.08(q, 0.5 H), 3.60(s, 3H), 1.17 (m, 6H).

### g) 11β-Fluor-3-methoxy-15β,16β-methylen-17-pregna-3,5-dien-21,17β-carbolacton

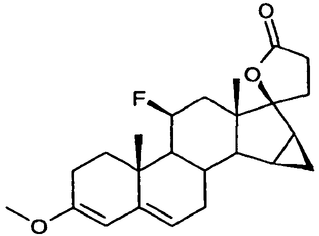

Es wurden 91 ml 1.6 M -Butylllitium-Lösung (in Hexan) bei - 50°C vorlegt und 14 g Allyl-tetramethylphosphorodiamidat, in 30 ml Tetrahydrofuran gelöst, zugetropft. Nach 30 min. Rühren bei -20 °C wurden 22 ml N,N,N,N-Tetramethylethandiamin eintragen und man ließ auf Raumtemperatur erwärmen. Eine Lösung von 15 g 11β-Fluor-3-methoxy-15β,16β-methylen-androsta-3,5-dien-17-on in 80 ml Tetrahydrofuran wurde zugegeben und 4 Stunden bei Raumtemperatur nachgerührt. Anschließend gab man gesättigte wässrige Ammmoniumchloridlösung zu und goß auf Wasser, extrahierte dreimal mit Ethylacetat, wusch mit Wasser und Kochsalzlösung neutral, trocknete über Natriumsulfat, und engte im Vakuum bei 40 °C ein. Nach Kristallisation aus Ethylacetat erhielt man 15,8 g 11β-Fluor-3-methoxy-15β,16β-methylen-17-pregna-3,5-dien-21,17β-carbolacton.
¹H-NMR (300 MHz, CDCl3): δ = 5.28-5.22 (m, 1.5H) 5.17 (m, 1H), 5.09 (q, 0.5 H), 3.63 (s, 3H), 1.20 (m, 6H), 0.53 (m, 1 H)

### h) 11β-Fluor-15β,16β-methylen-3-oxo-17.pregna-4,6-dien-21,17β-carbolacton

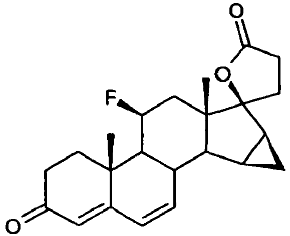

Zu einer Suspension von 13.5 g 11β-Fluor-3-methoxy-15β,16β-methylen-17-pregna-3,5-dien-21,17β-carbolacton in 150 ml 1-Methyl-2-pyrrolidon wurden nacheinander bei 0 °C 14.5 ml einer 10%igen Natriumacetatlösung sowie 5.11 g 1,3-Dibrom-5,5-dimethylhydantoin portionsweise zugesetzt. Anschließend wurde 0.5 Stunden bei 0°C (Eisbad) gerührt, mit 4.86 g Lithiumbromid sowie 4.27 g Lithiumcarbonat versetzt, und 3.5 Stunden bei 100°C Badtemperatur gerührt. Anschließend goß man in Eiswasser / Kochsalz und filtrierte den Niederschlag ab. Nach Chromatografie an Kieselgel 60 (Elution mit Hexan / Ethylacetat 1:1) erhielt man 9,1 g 11β-Fluor-15β,16β-methylen-3-oxo-17-pregna-4,6-dien-21,17β-carbolacton.
¹H-NMR (600 MHz, CDCl3): δ = 6.41 (m, 1H), 6.22 (m, 1 H), 5.68 (s, 1H), 5.11 (dq, 1 H), 1.31 (d, 3H), 1.21(d, 3H), 0.60 (m, 1H).

### i) 6β,7β;15β,16β-Dimethylen-11β-fluor-3-oxo-17-pregn-4-en-21,17β-carbolacton

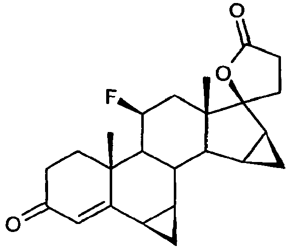

Zu einer Lösung von 13.41 g Trimethylsulfoxoniumiodid in 250 ml trockenem DMSO gab man bei Raumtempemperatur portionsweise 2.39 g Natriumhydrid (60% in Mineraloel) und ließ nach beendeter Zugabe 3 Stunden bei Raumtemperatur rühren. Anschließend trug man 8.38 g 11β-Fluor-15β,16β-methylen-3-oxo-17-pregna-4,6-dien-21,17β-carbolacton ein und rührte 6 Stunden bei Raumtemperatur. Danach goß man auf Wasser, extrahierte dreimal mit Ethylacetat, wusch mit Wasser und Kochsalzlösung neutral, trocknete über Natriumsulfat und engte im Vakuum bei 40 °C ein. Zur Reinigung wurde an Kieselgel 60 chromatografiert (Elution mit Hexan Ethylacetat 1:4). Man erhielt als Fraktion A 2,6 g 6β,7β;15β,16β-Dimethylen-11β-fluor-3-oxo-17-pregn-4-en-21,17β-carbolacton.
MS (EI): m/z = 384 (M⁺), 349, 273, 260;
¹H-NMR (600 MHz, CDCl3): δ = 5.99 (s, 1 H), 5.07 (d(br), 1 H), 2.69-2.61 (m, 2H), 2.53 (m, 1 H), 2.43 (d(br), 1 H), 2.35 (m, 1 H), 2.27 (m, 1 H), 2.17-2.10 (m, 2H), 2.02-1.95 (m, 2H), 1.83 (m, 1H), 1.68-1.62 (m, 2H), 1.61-1.52 (m, 2H), 1.49 (m, 1 H), 1.40 (m, 1 H), 1.29 (d, 3H), 1.25 (m, 1 H), 1.21 (m, 1 H), 1.15 (d, 3H), 1.04 (m, 1 H), 0.59 (m, 1 H)

### Alternative Synthesemethode des Beispiels 2:

### c') 15β,16β-Methylen-androsta-4,9(11)-dien-3,17-dion und e') 11β-Fluor-15β,16β-methylen-androst-4-en-3,17-dion

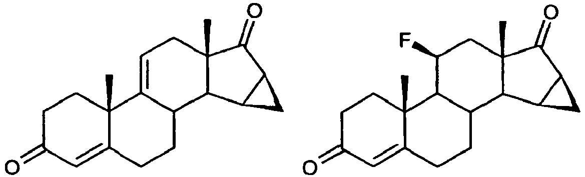

Eine Lösung von 630 mg 11 α-Hydroxy-15β,16β-methylen-androst-4-en-3,17-dion in 16 ml Tetrahydrofuran wurde bei 0 °C tropfenweise mit 0.47 ml 1,8-Diazabicyclo-[5.4.0]-undec-7-en (1,5-5) so versetzt, dass die Innentemperatur 5 °C nicht überstieg. Anschließend wurde 30 min bei 0 °C gerührt, tropfenweise mit 0.55 ml Perfluorbutan-1-sulfonsäurefluorid so versetzt, dass die Innentemperatur 5 °C nicht überstieg und weitere 1.5 Stunden bei 0 °C gerührt. Anschliessend verdünnte man mit Ethylacetat, wusch mit 2M Schwefelsäure, gesättigter Natriumhydrogencarbonatlösung und Wasser, trocknete über Natriumsulfat und engte im Vakuum bei 40 °C ein. Durch Chromatographie an Kieselgel erhielt man nach Elution mit Hexan / Ethylacetat (1:1) 15β,16β-Methylenandrosta-4,9(11 1)-dien-3,17-dion als Fraktion 1.
1 H-NMR (600 MHz, CDCI3): δ = 5.79(m, 1 H), 5.55(m, 1 H), 1.85(m,1 H), 1.65(m,1 H), 1.37(s, 3H), 1.12-1.33 (2m, 2H), 1.00 (s, 3H)

Als Fraktion 2 wurde 11β-Fluor-15β, 16β-methylen-androst-4-en-3,17-dion isoliert. ¹H-NMR (600 MHz, CDCl3): δ = 5.73(m, 1 H), 5.28(dq, 1 H), 1.395(d, 3H), 1.175 (d, 3H).

### Beispiel 3

### 6a,7a;1 5β,16β-Dimethylen-11β-fluor-3-oxo-17-pregn-4-en-21,17β-carbolacton:

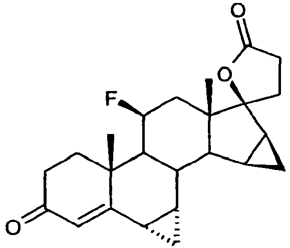

Als Fraktion B des Beispiels 2 erhielt man 0,37 g 6α,7α;15β,16β-Dimethylen-11β-fluor-3-oxo-17-pregn-4-en-21,17β-carbolacton.
MS (EI): m/z = 384 (M⁺);
¹H-NMR (600 MHz, CDCl3): δ = 5.94 (s, 1 H), 5.08 (d(br), 1 H), 1.35 (s, 3H), 1.25 (m, 1 H), 1.21 (m,1H), 1.20 (d, 3H), 1.00 (m, 1 H), 0.76 (ddd, 1 H), 0.54 (m, 1 H) 0.48 (m, 1 H)

### Alternative Synthesemethoden des Beispiels 1:

### 1. Variante

### a. 11α-Hydroxy-3-methoxy-15β,16β-methylen-androst-3,5-dien-17-on

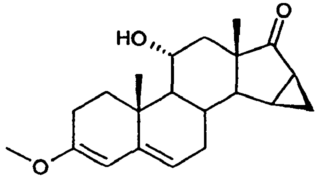

In eine Suspension von 27 g 11α-Hydroxy-15β,16β-methylen-androst-4-en-3,17-dion in 422 ml 2,2-Dimethoxypropan wurden 3.2 g Pyridintosylat eintragen. Dann wurde 18 h bei 100°C Badtemperatur gerührt. Nach dem Abkühlen auf Raumtemperatur gab man 10 ml Triethylamin zu und engte im Vakuum zur Trockne ein. Der Rückstand wurde mit 60 ml Methanol ausgerührt und abgesaugt. Man erhielt 14,3 g 11α-Hydroxy-3-methoxy-15β,16β-methylen-androst-3,5-dien-17-on.
¹H-NMR (400 MHz, CDCl3): δ = 5.33 (d, breit, J=3.8Hz, 1H), 5.14 (s, breit, 1 H), 4.07 (m, 1H), 3.58 (s, 3H), 1.79 (m, 1H), 1.13 (s, 3H), 1.02 (s, 3H)

### b) 11α-Hydroxy-3-methoxy-15β,16β-methylen-17-pregna-3,5-dien-21,17β-carbolacton

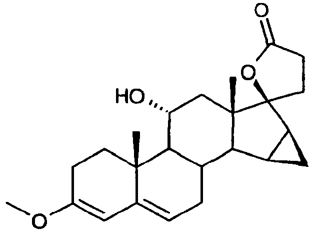

Es wurden 66,6 ml 1.6 M -Butylllitium-Lösung (in Hexan) bei - 50°C vorlegt und 10,24 g Allyl-tetramethylphosphorodiamidat, in 13 ml Tetrahydrofuran gelöst, zugetropft. Nach 30 min. Rühren bei -20 °C wurden 16 ml N,N,N,N-Tetramethylethandiamin eintragen und anschließend eine Lösung von 5 g 11α-Hydroxy-3-methoxy-15β,16β-methylen-androst-3,5-dien-17-on in 33,5 ml Tetrahydrofuran zugetropft. Es wurde auf Raumtemperatur erwärmt und 30 Minuten nachgerührt. Anschließend gab man 25 ml gesättigte wässrige Ammmoniumchloridlösung zu und goß auf Wasser, extrahierte dreimal mit Ethylacetat, wusch mit Wasser und Kochsalzlösung neutral, trocknete über Natriumsulfat und engte im Vakuum ein. Nach Kristallisation aus Düsopropylether erhielt man 2,85 g 11α-Hydroxy-3-methoxy-15β,16β-methylen-17-pregna-3,5-dien-21,17β-carbolacton.
¹H-NMR (400 MHz, CDCl3): δ = 5.31 (d, breit, J=4.0Hz, 1H), 5.14 (s, breit, 1H), 4.06 (m, 1H), 3.58 (s, 3H), 1.14 (s, 3H), 1.02 (s, 3H), 0.46 (m, 1 H)

### c) 11α-Hydroxy-15β,16β-methylen-3-oxo-17-pregna-4,6-dien-21,17β-carbolacton

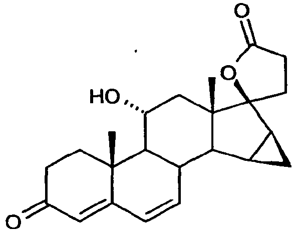

Zu einer Suspension von 13,5 g 11α-hydroxy-3-methoxy-15β,16β-methylen-17-pregna-3,5,9(11)-trien-21,17β-carbolacton in 144 ml 1-Methyl-2-pyrrolidon wurden nacheinander bei 0 °C 14,8 ml einer 10%igen Natriumacetatlösung sowie 4 g 1,3-Dibrom-5,5-dimethylhydantoin portionsweise zugesetzt. Anschließend wurde 0.5 Stunden bei 0°C (Eisbad) gerührt, mit 4,88 g Lithiumbromid sowie 4,31 g Lithiumcarbonat versetzt und 3 Stunden bei 80°C Badtemperatur gerührt. Anschließend goß man in eiskalte gesättigte wässrige Natriumchloridlösung, extrahierte mit Ethylacetat, wusch die organische Phase mit Wasser und gesättigter wässriger Natriumchloridlösung, trocknete über Natriumsulfat, filtrierte und engte das Filtrat zur Trockne ein. Es wurden 12,8 g 11 α-Hydroxy-15β,16β-methylen-3-oxo-17-pregna-4,6-dien -21,17β-carbolacton als Rohprodukt erhalten. Für analytische Zwecke wurde eine Probe an Kieselgel mit einem Gemisch aus Hexan und Ethylacetat chromatographiert.
¹H-NMR (400 MHz, CDCl3): δ = 6.34 (d, breit, J=9.6Hz, 1H), 6.20 (d, breit, J=9.6Hz, 1H), 5.71 (s, breit, 1 H), 4.05 (m, 1 H), 1.95 (m, 1 H), 1.85 (m, 1 H), 1.29 (m, 1 H), 1.25 (s, 3H), 1.09 (s, 3H), 0.57 (m, 1 H)

### d) 11α-Mesyloxy-15β,16β-methylen-3-oxo-17-pregna-4,6-dien-21,17β-carbolacton

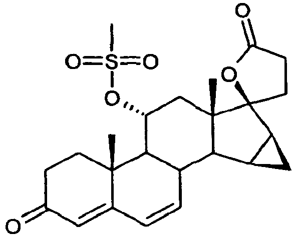

12,8 g 11α-Hydroxy-15β,16β-methylen-3-oxo-17-pregna-4,6-dien -21,17β-carbolacton wurden in 113 ml Pyridin gelöst. Anschließend wurden 10,91 ml Methansulfonsäurechlorid zugetropt. Es wurde 90 Minuten bei Raumtemperatur gerührt und auf 1,5 I Eiswasser gegossen. Nach zweistündigem Rühren wurde abgesaugt, der Filterkuchen getrocknet und selbiger an Kieselgel mit einem Gemisch aus Hexan und Ethylacetat chromatographiert. Es wurden 5,4 g 11α-Mesyloxy-15β,16β-methylen-3-oxo-17-pregna-4,6-dien -21,17β-carbolacton erhalten.
¹H-NMR (400 MHz, CDCl3): δ = 6.33 (d, breit, J=9.6Hz, 1H), 6.23 (d, breit, J=9.6Hz, 1 H), 5.74 (s, breit, 1H), 5.10 (m, 1 H), 3.01 (s, 3H), 1.56 (m, 1H), 1.45 (m, 1H), 1.30 (s, 3H), 1.14 (s, 3H), 0.59 (m, 1 H)

### e) 15β,16β-methylen-3-oxo-17-pregna-4,6,9(11)-trien-21,17β-carbolacton

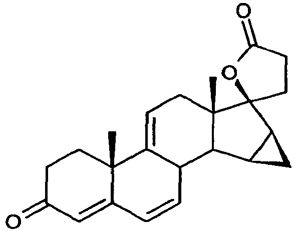

14,8 ml Essigsäure, 0,16 ml Acetanhydrid und 2,44 g Natriumacetat wurden bei 90°C gerührt, bis das Natriumacetat in Lösung gegangen ist. Zu dieser Lösung wurden 5,3 g 11α-Mesyloxy-15β,16β-methylen-3-oxo-17-pregna-4,6-dien -21,17β-carbolacton gegeben. Nachdem 5 Stunden bei 100°C gerührt worden war, wurde auf Eiswasser gegossen und dreimal mit Ethylacetat extrahiert. Nach Waschen der organischen Phase mit Wasser, gesättigter wässriger Natriumchloridlösung und Trocknung über Magnesiumsulfat wurde filtriert und das Filtrat eingeengt. Nach Chromatographie an Kieselgel mit einem Gemisch aus Hexan und Ethylacetat wurden 2,12 g 15β, 16β-methylen-3-Oxo-17-pregna-4,6,9(11)-trien -21,17β-carbolacton erhalten.
¹H-NMR (300 MHz, CDCl3): δ = 6.36 (d, breit, J=9.6Hz, 1H), 6.24 (d, breit, J=9.6Hz, 1H), 5.72 (s, breit, 1H), 5.48 (m, 1 H), 3.09 (d, breit, J=11.7Hz, 1 H), 1.84 (m, 1H), 1.47 (m, 1H), 1.38 (m, 1H), 1.32 (s, 3H), 1.03 (s, 3H), 0.59 (m, 1H)

### f) 6β,7β;15β,16β-Dimethylen-3-oxo-17-pregna-4,9(11)-dien-21,17β-carbolacton

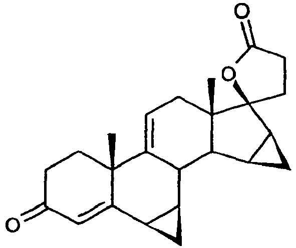

Zu einer Lösung von 0,52 g Trimethylsulfoxoniumiodid in 4 ml trockenem DMSO gab man bei Raumtempemperatur portionsweise 0,09 g Natriumhydrid (60% in Mineraloel) und ließ nach beendeter Zugabe 2 Stunden bei Raumtemperatur rühren. Anschließend trug man bei 0°C 0,2 g 15β,16β-methylen-3-oxo-17-pregna-4,6,9(11)-trien -21,17β-carbolacton ein und rührte 2,5 Stunden bei Raumtemperatur nach. Danach wurde der Ansatz in 100 ml Schwefelsäure (8 volumenprozentig) eingerührt und mit Ethylacetat extrahiert. Die organische Phase wurde nacheinander mit Wasser und gesättigter Natriumchloridlösung gewaschen, über Natriumsulfat getrocknet und filtriert. Nach Einengen im Vakuum und Chromatographie an Kieselgel mit einem Eluenten aus Ethylacetat und Hexan erhielt man 30 mg 6β,7β; 15β, 16β-Dimethylen-3-oxo-17-pregna-4,9(11)-dien-21,17β-carbolacton.

Spektroskopische Daten vgl. Beispiel 1 b.

### 2. Variante

### a. 3-Methoxy-15β,16β-methylen-and rost-3, 5,9(11)-trien-17-on

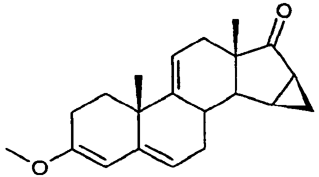

In eine Suspension von 6,4 g 15β,16β-Methylen-androst-4,6,9(11)-trien-3,17-dion in 106 ml 2,2-Dimethoxypropan wurden 0,8 g Pyridintosylat eintragen. Dann wurde 6 h bei 100°C Badtemperatur gerührt. Nach dem Abkühlen auf Raumtemperatur gab man 5 ml Pyridin zu und engte nach 5 Minuten im Vakuum zur Trockne ein. Der Rückstand wurde mit 130 ml Methanol ausgerührt und abgesaugt. Man erhielt 4,15 g 3-Methoxy-15β,16β-methylen-androst-3,5,9(11)-trien-17-on.
¹H-NMR (400 MHz, CDCl3): δ = 5.47 (s, breit, 1H), 5.33 (s, breit, 1 H), 5.19 (s, breit, 1 H), 3.59 (s, 3H), 2.70 (m, 2H), 2.38 (m, 1 H), 1.83 (m, 1 H), 1.66 (m, 1 H), 1.15 (s, 3H), 0.99 (s, 3H)

### b) 3-Methoxy-15β,16β-methylen-17-pregna-3,5,9(11)-trien-21,17β-carbolacton

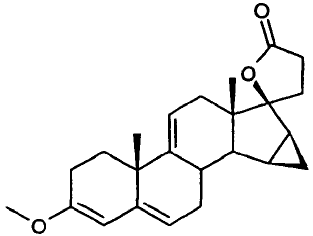

Es wurden 42,2 ml 1.6 M -Butylllitium-Lösung (in Hexan) bei - 50°C vorgelegt und 6,51 g Allyl-tetramethylphosphorodiamidat, in 11,4 ml Tetrahydrofuran gelöst, zugetropft. Nach 30 min. Rühren bei -20 °C wurden 10,21 ml N,N,N,N-Tetramethylethandiamin eintragen und anschließend wurde eine Lösung von 4,14 g 3-Methoxy-15β,16β-methylenandrost-3,5,9(11)-trien-17-on in 29,4 ml Tetrahydrofuran zugetropft. Es wurde auf Raumtemperatur erwärmt und 30 Minuten nachgerührt. Anschließend gab man 21 ml gesättigte wässrige Ammmoniumchloridlösung zu und goß auf Wasser, extrahierte dreimal mit Ethylacetat, wusch mit Wasser und Kochsalzlösung neutral, trocknete über Natriumsulfat und engte im Vakuum ein, wobei Kristallisation einsetzte. Nach Absaugen vom Restlösemittel wurden 3,14 g 3-Methoxy-15β,16β-methylen-3,5,9(11)-trien-21,17β-carbolacton erhalten.
¹H-NMR (300 MHz, CDCl3): ö = 5.45 (s, breit, 1 H), 5.32 (s, breit, 1 H), 5.18 (s, breit, 1 H), 3.59 (s, 3H), 1.15 (s, 3H), 0.96 (s, 3H)

### c) 15β,16β-methylen-3-oxo-17-pregna-4,6,9(11)-trien-21,17β-carbolacton

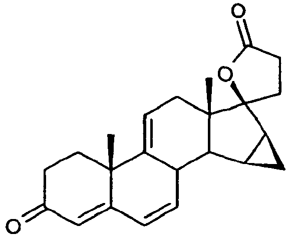

Zu einer Suspension von 2,2 g 3-Methoxy-15β,16β-methylen-17-pregna-3,5,9(11)-trien-21,17β-carbolacton in 35 ml 1-Methyl-2-pyrrolidon wurden nacheinander bei 0 °C 1,7 ml einer 10%igen Natriumacetatlösung sowie 0,6 g 1,3-Dibrom-5,5-dimethylhydantoin portionsweise zugesetzt. Anschließend wurde 0.5 Stunden bei 0°C (Eisbad) gerührt, mit 0,83 g Lithiumbromid sowie 0,74 g Lithiumcarbonat versetzt, und 3.5 Stunden bei 100°C Badtemperatur gerührt. Anschließend goß man in Eiswasser / Kochsalz und filtrierte den Niederschlag ab. Nach Chromatografie an Kieselgel 60 (Elution mit Hexan / Ethylacetat 1:1) erhielt man 1,2 g 15β,16β-methylen-3-oxo-17-pregna-4,6,9(11)-trien-21,17β-carbolacton.

Spektroskopische Daten vergleiche 1. Variante e

### d) 6β,7β;15β,16β-Dimethylen-3-oxo-17-pregna-4,9(11)-dien 21,17β-carbolacton

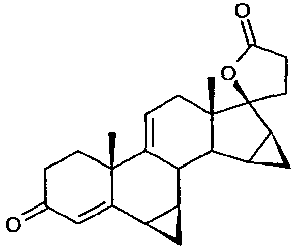

Durchführung und Aufarbeitung vgl. 1. Variante f.

## Patentansprüche

1. Pregn-4-en-21,17-carbolactone der allgemeinen Formel I 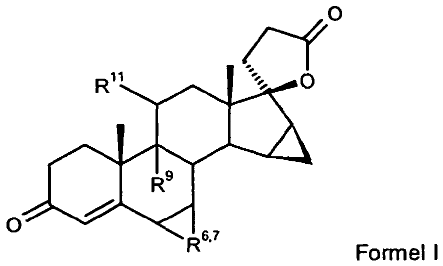 worin
R^{6.7} ein α- oder β-ständiges Methylen und
R⁹ ein Wasserstoffatom und R¹¹ ein Brom-. Chlor- oder Fluoratom oder
R⁹ und R¹¹ gemeinsam eine Bindung bedeuten.

2. Pregn-4-en-21,17-carbolactone nach Anspruch 1, **dadurch gekennzeichnet, dass** sich R⁹ in der α-Position befindet.

3. Pregn-4-en-21,17-carbolactone nach Anspruch 1, **dadurch gekennzeichnet, dass** sich R¹¹ in der β-Stellung befindet.

4. Pregn-4-en-21,17-carbolactone nach Anspruch 1, **dadurch gekennzeichnet, dass** das Halogenatom R¹¹ ein Fluor- oder Chloratom ist.

5. Pregn-4-en-21,17-carbolactone nach Anspruch 4, **dadurch gekennzeichnet, dass** das Halogenatom R¹¹ ein Fluoratom ist.

6. 6β,7β;15β,16β-Dimethylen-3-oxo-17-pregna-4,9(11)-dien-21,17β-carbolacton nach Anspruch 1.

7. Pregn-4-en-21,17-carbolactone nach Anspruch 1, nämlich 11β-Chlor-6β,7β;15β,16β-dimethylen-3-oxo-17-pregn-4-en-21,17β-carbolacton 6β,7β;15β,16β-Dimethylen-11β-fluor-3-oxo-17-pregn-4-en-21,17β-carbolacton 6α,7α;15β,16β-Dimethylen-11β-fluor-3-oxo-17-pregn-4-en-21,17β-carbolacton.

8. Pharmazeutische Präparate enthaltend mindestens eine Verbindung der allgemeinen Formel I gemaß Anspruch 1 sowie einen pharmazeutisch unbedenklichen Träger.

9. Pharmazeutische Präparate nach Anspruch 8 enthaltend 6β,7β;15β,16β-Dimethylen-3-oxo-17-pregna-4,9(11)-dien-21,17β-carbolacton.

10. Pharmazeutische Präparate nach Anspruch 8 enthaltend 6β,7β;15β,16β-Dimethylen-11β-fluor-3-oxo-17-pregn-4-en-21,17β-carbolacton.

11. Pharmazeutische Präparate nach Anspruch 8, 9 oder 10 außerdem enthaltend mindestens ein Estrogen.

12. Pharmazeutische Präparate nach Anspruch 11 enthaltend Ethinylestradiol.

13. Pharmazeutische Präparate nach Anspruch 11, enthaltend ein natürliches Estrogen.

14. Pharmazeutische Präparate nach Anspruch 13, enthaltend Estradiol.

15. Pharmazeutische Präparate nach Anspruch 13, enthaltend Estradiolvalerat.

16. Pharmazeutische Präparate nach Anspruch 13, enthaltend mindestens ein konjugiertes Estrogen.

17. 11α-Hydroxy-15β,16β-methylen-androst-4-en-3,17-dion als Ausgangsverbindung zur Herstellung der Verbindungen der allgemeinen Formel I.

18. Verfahren zur Herstellung von 11α-Hydroxy-15β,16β-methylen-androst-4-en-3,17-dion, **dadurch gekennzeichnet, daß** 15β,16β-Methylen-androst-4-en-3,17-dion in einem Fermentationsbehälter mit Mikroorganismen der Spezies *Absidia sp., Acremonium sp., Ascochyta sp., Aspergillus sp., Bacillus sp., Beauveria sp., Botryodipoldia sp., Caldariomyces sp., Calonectria sp., Colletotrichum sp., Curvularia sp., Fusarium sp., Gibberella sp., Gloeosporium sp., Glomerella sp., Gnomonia sp., Haplosporella sp., Helicostylum sp., Helminthosporium sp., Metarhizium sp., Mucor sp., Nigrospora sp., Rhizopus sp., Sporotrichum sp., Syncephalastrum sp.,* und *Wojnowicia s.p* hydroxyliert wird.

19. Verfahren nach Anspruch 18, **dadurch gekennzeichnet, dass** mit *Absidia orchidis, Absidia coerulea, Acremonium strictum, Ascochyta clematidina, Aspergillus alliaceus, Aspergillus awamori, Aspergillus fischeri, Aspergillus flavus, Aspergillus malignus, Aspergillus melleus, Aspergillus nidualans, Aspergillus niger, Aspergillus ochraceus, Aspergillus variecolor, Bacillus megaterium, Beauveria bassiana, Beauveria tenella, Botryodiplodia malorum, Caldariomyces fumago, Calonectria decora, Colletotrichum phomoides, Curvularia lunata, Fusarium oxysporium, Fusarium solani, Gibberella zeae, Glomerella cingulata, Gloeosporium fructigenum, Gloeosporium higgensianum, Gloeosporium kaki, Gloeosporium lacticolor, Gloeosporium olivarum, Glomerella fusaroides, Gnomonia cingulata, Haplosporella hesperedica, Helminthosporium sp., Helicostylum piriforme, Metarhizium anisopliae, Mucor plumbeus, Mucor spinosus, Nigrospora sphaerica, Rhizopus arrhizus, Rhizopus cohnii, Rhizopus delemar, Rhizopus japonicus, Rhizopus kazaensis, Rhizopus microsporus, Rhizopus oryzae, Rhizopus shanghaiensis, Rhizopus stolonifer, Rhizopus tritici, Sporotrichum sulfurescens, Syncephalastrum racemosum, Wojnowicia* graminis *und Wojnowicia hirta* hydroxyliert wird.

20. Verfahren nach Anspruch 19, **dadurch gekennzeichnet, dass** mit *Absidia orchidis* (ATCC 6647), *Acremonium strictum* (NRRL 5759), *Ascochyta clematidina* (CBS), *Aspergillus alliaceus* (ATCC 10060), *Aspergillus awamori* (CBS), *Aspergillus fischeri* (ATCC 1020), *Aspergillus malignus* (IMI 16061), *Aspergillus melleus* (CBS), *Aspergillus nidualans* (ATCC 11267), *Aspergillus niger* (ATCC 9142, ATCC 11394), *Aspergillus ochraceus* (NRRL405, NRRL 410, CBS 13252, ATCC 46504), *Aspergillus variecolor* (ATCC 10067), *Bacillus megaterium* (ATCC 13368), *Beauveria bassiana* (IFO 5838, ATCC 13144, IFO 4848, CBS 11025, CBS 12736, ATCC 7159), *Botryodiplodia malorum* (CBS 13450), *Caldariomyces fumago* (ATCC 16373), *Calonectria decora* (ATCC 14767), *Curvularia lunata* (IX 3, NRRL 2380), *Fusarium solani* (ATCC 12823), *Fusarium oxysporum* (ATCC 7808), *Gibberella zeae* (CBS 4474), *Glomereila cingulata* (ATCC 12097, ATCC 10534, CBS 23849, CBS 23749, ATCC 16646, IFO 6459, IFO 6425, IFO 6470, ATCC 15093, ATCC 10529, IFO 5257, ATCC 56596, ATCC 64682), *Glomereita fusaroides* (ATCC 9552), *Gnomonia cingulata* (CBS 15226), *Haplosporeila hesperedica* (CBS 20837), *Helicostylum piriforme (A TCC* 8992), *Helminthosporium sp.* (NRRL 4671), Me*tarhizium anisopliae* (IFO 5940), *Mucor plumbeus* (CBS 29563), *Nigrospora sphaerica* (ATCC 12772), *Rhizopus arrhizus* (ATCC 11145), *Rhizopus oryzae* (ATCC 4858, ATCC 34102, CBS 32947), *Rhizopus stolonifer* (ATCC 15441), *Syncephalastrum racemosum* (IFO 4827) und *Wojnowicia graminis* (CBS 89168) hydroxyliert wird.

## Claims

1. Pregn-4-ene-21,17-carbolactones of the general formula I 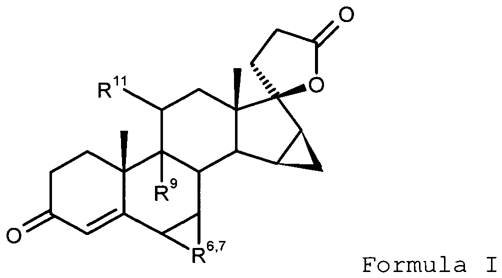 in which
R^{6,7} is an α- or α-methylene and
R⁹ is a hydrogen atom and R¹¹ is a bromine, chlorine or fluorine atom or
R⁹ and R¹¹ together are a bond.

2. Pregn-4-ene-21,17-carbolactones according to Claim 1, **characterized in that** R⁹ is located in the □ position.

3. Pregn-4-ene-21,17-carbolactones according to Claim 1, **characterized in that** R¹¹ is located in the β position.

4. Pregn-4-ene-21,17-carbolactones according to Claim 1, **characterized in that** the halogen atom R¹¹ is a fluorine or chlorine atom.

5. Pregn-4-ene-21,17-carbolactones according to Claim 4, **characterized in that** the halogen atom R¹¹ is a fluorine atom.

6. 6β,7β;15β,16β-Dimethylene-3-oxo-17-pregna-4,9(11)-diene-21,17β-carbolactone according to Claim 1.

7. Pregn-4-ene-21,17-carbolactones according to Claim 1, specifically 11β-chloro-6β,7β;15β,16β-dimethylene-3-oxo-17-pregn-4-ene-21,17β-carbolactone 6β,7β;15β,16β-dimethylene-11β-fluoro-3-oxo-17-pregn-4-ene-21,17β-carbolactone 6α,7α;15β,16β-dimethylene-11β-fluoro-3-oxo-17-pregn-4-ene-21,17β-carbolactone.

8. Pharmaceutical products comprising at least one compound of the general formula I according to Claim 1, and a pharmaceutically acceptable carrier.

9. Pharmaceutical products according to Claim 8, comprising 6β,7β;15β,16β-dimethylene-3-oxo-17-pregna-4,9(11)-diene-21,17β-carbolactone.

10. Pharmaceutical products according to Claim 8, comprising 6β,7β;15β,16β-dimethylene-11β-fluoro-3-oxo-17-pregn-4-ene-21,17β-carbolactone.

11. Pharmaceutical products according to Claim 8, 9 or 10 additionally comprising at least one oestrogen.

12. Pharmaceutical products according to Claim 11, comprising ethinylestradiol.

13. Pharmaceutical products according to Claim 11, comprising a natural oestrogen.

14. Pharmaceutical products according to Claim 13, comprising oestradiol.

15. Pharmaceutical products according to Claim 13, comprising oestradiol valerate.

16. Pharmaceutical products according to Claim 13, comprising at least one conjugated oestrogen.

17. 11α-Hydroxy-15β,16β-methyleneandrost-4-ene-3,17-dione as starting compound for preparing the compounds of the general formula I.

18. Process for preparing 11α-hydroxy-15β,16β-methyleneandrost-4-ene-3,17-dione, **characterized in that** 15β,16β-methyleneandrost-4-ene-3,17-dione is hydroxylated in a fermentor with microorganisms of the species *Absidia sp., Acremonium sp., Ascochyta sp., Aspergillus sp., Bacillus sp., Beauveria sp., Botryodiplodia sp., Caldariomyces sp., Calonectria sp., Colletotrichum sp., Curvularia sp., Fusarium sp., Gibberella sp., Gloeosporium sp., Glomerella sp., Gnomonia sp., Haplosporella sp., Helicostylum sp., Helminthosporium sp., Metarhizium sp., Mucor sp., Nigrospora sp., Rhizopus sp., Sporotrichum sp., Syncephalastrum sp.,* and *Wojnowicia s.p.*

19. Process according to Claim 18, **characterized in that** hydroxylation is carried out with *Absidia orchidis, Absidia coerulea, Acremonium strictum, Ascochyta clematidina, Aspergillus alliaceus, Aspergillus awamori, Aspergillus fischeri, Aspergillus flavus, Aspergillus malignus, Aspergillus melleus, Aspergillus nidulans, Aspergillus niger, Aspergillus ochraceus, Aspergillus variecolor, Bacillus megaterium, Beauveria bassiana, Beauveria tenella, Botryodiplodia malorum, Caldariomyces fumago, Calonectria decora, Colletotrichum phomoides, Curvularia lunata, Fusarium oxysporum, Fusarium solani, Gibberella zeae, Glomerella cingulata, Gloeosporium fructigenum, Gloeosporium higgensianum, Gloeosporium kaki, Gloeosporium lacticolor, Gloeosporium olivarum, Glomerella fusaroides, Gnomonia cingulata, Haplosporella hesperedica, Helminthosporium sp., Helicostylum piriforme, Metarhizium anisopliae, Mucor plumbeus, Mucor spinosus, Nigrospora sphaerica, Rhizopus arrhizus, Rhizopus cohnii, Rhizopus delemar, Rhizopus japonicus, Rhizopus kazaensis, Rhizopus microsporus, Rhizopus oryzae, Rhizopus shanghaiensis, Rhizopus stolonifer, Rhizopus tritici, Sporotrichum sulfurescens, Syncephalastrum racemosum, Wojnowicia* graminis and *Wojnowicia hirta.*

20. Process according to Claim 19, **characterized in that** hydroxylation is carried out with *Absidia orchidis* (ATCC 6647), *Acremonium strictum* (NRRL 5759), *Ascochyta clematidina* (CBS), *Aspergillus alliaceus* (ATCC 10060), *Aspergillus awamori* (CBS), *Aspergillus fischeri* (ATCC 1020)*, Aspergillus malignus* (IMI 16061), *Aspergillus melleus* (CBS), *Aspergillus nidulans* (ATCC 11267), *Aspergillus niger* (ATCC 9142, ATCC 11394), *Aspergillus ochraceus* (NRRL405, NRRL 410, CBS 13252, ATCC 46504), *Aspergillus variecolor* (ATCC 10067), *Bacillus megaterium* (ATCC 13368), *Beauveria bassiana* (IFO 5838, ATCC 13144, IFO 4848, CBS 11025, CBS 12736, ATCC 7159), *Botryodiplodia malorum* (CBS 13450), *Caldariomyces fumago* (ATCC 16373), *Calonectria decora* (ATCC 14767), *Curvularia lunata* (IX 3, NRRL 2380), *Fusarium solani* (ATCC 12823), *Fusarium oxysporum* (ATCC 7808), *Gibberella zeae* (CBS 4474), *Glomerella cingulata* (ATCC 12097, ATCC 10534, CBS 23849, CBS 23749, ATCC 16646, IFO 6459, IFO 6425, IFO 6470, ATCC 15093, ATCC 10529, IFO 5257, ATCC 56596, ATCC 64682), *Glomerella fusaroides* (ATCC 9552), *Gnomonia cingulata* (CBS 15226), *Haplosporella hesperedica* (CBS 20837), *Helicostylum piriforme (ATCC 8992), Helminthosporium sp.* (NRRL 4671), *Metarhizium anisopliae* (IFO 5940), *Mucor plumbeus* (CBS 29563), *Nigrospora sphaerica* (ATCC 12772), *Rhizopus arrhizus* (ATCC 11145), *Rhizopus oryzae* (ATCC 4858, ATCC 34102, CBS 32947), *Rhizopus stolonifer* (ATCC 15441), *Syncephalastrum racemosum* (IFO 4827) and *Wojnowicia graminis* (CBS 89168).

## Revendications

1. Prégn-4-ène-21,17-carbolactones de formule générale 1 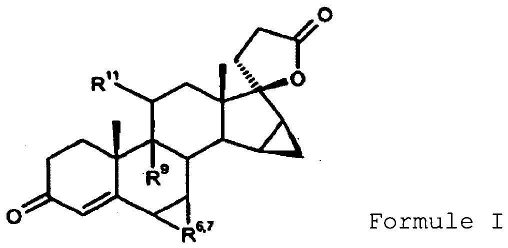 dans laquelle
R^{6.7} représente un groupe méthylène en position α ou β et
R⁹ représente un atome d'hydrogène et R¹¹ représente un atome de brome, de chlore ou de fluor ou
R⁹ et R¹¹ représentent ensemble une liaison.

2. Prégn-4-ène-21,17-carbolactones selon la revendication 1, **caractérisées en ce que** R⁹ se trouve en la position α.

3. Prégn-4-ène-21,17-carbolactones selon la revendication 1, **caractérisées en ce que** R¹¹ se trouve en la position β.

4. Prégn-4-ène-21,17-carbolactones selon la revendication 1, **caractérisées en ce que** l'atome d'halogène R¹¹ est un atome de fluor ou de chlore.

5. Prégn-4-ène-21,17-carbolactones selon la revendication 4, **caractérisées en ce que** l'atome d'halogène R¹¹ est un atome de fluor.

6. 6β,7β;15β,16β-diméthylène-3-oxo-17-prégna-4,9(11)-diène-21,17β-carbolactone selon la revendication 1.

7. Prégn-4-ène-21,17-carbolactones selon la revendication 1, à savoir 11β-chloro-6β,7β;15β,16β-diméthylène-3-oxo-17-prégn-4-ène-21,17β-carbolactone 6β,7β;15β,16β-dimethylène-11β-fluoro-3-oxo-17-prégn-4-ène-21,17β-carbolactone 6α,7α;15β,16β-dimethylène-11β-fluoro-3-oxo-17-prégn-4-ène-21,17β-carbolactone.

8. Préparations pharmaceutiques contenant au moins un composé de formule générale I selon la revendication 1 ainsi qu'un véhicule pharmaceutiquement acceptable.

9. Préparations pharmaceutiques selon la revendication 8, contenant de la 6β,7β;15β,16β-diméthylène-3-oxo-17-prégna-4,9(11)-diène-21,17β-carbolactone.

10. Préparations pharmaceutiques selon la revendication 8, contenant de la 6β,7β;15β,16β-diméthylène-11β-fluoro-3-oxo-17-prégn-4-ène-21,17β-carbolactone.

11. Préparations pharmaceutiques selon la revendication 8, 9 ou 10, en outre contenant au moins un oestrogène.

12. Préparations pharmaceutiques selon la revendication 11, contenant de l'éthinylestradiol.

13. Préparations pharmaceutiques selon la revendication 11, contenant un oestrogène naturel.

14. Préparations pharmaceutiques selon la revendication 13, contenant de l'estradiol.

15. Préparations pharmaceutiques selon la revendication 13, contenant du valérate d'estradiol.

16. Préparations pharmaceutiques selon la revendication 13, contenant au moins un oestrogène conjugué.

17. 11α-hydroxy-15β,16β-méthylène-androst-4-ène-3,17-dione en tant que composé de départ pour la préparation des composés de formule générale I.

18. Procédé pour la préparation de α-hydroxy-15β,16β-méthylène-androst-4-ène-3,17-dione, **caractérisé en ce qu'**on soumet à une hydroxylation de la 15β,16β-méthylène-androst-4-ène-3,17-dione dans un récipient de fermentation avec des micro-organismes appartenant aux espèces *Absidia sp., Acremonium sp., Ascochyta sp., Aspergillus sp., Bacillus sp., Beauveria sp., Botryodiplodia sp., Caldariomyces sp., Calonectria sp., Colletotrichum sp., Curvularia sp., Fusarium sp., Gibberella sp., Gloeosporium sp., Glomerella sp., Gnomonia sp., Haplosporella sp., Helicostylum sp., Helminthosporium sp., Metarhizium sp., Mucor sp., Nigrospora sp., Rhizopus sp., Sporotrichum sp., Syncephalastrum sp.* et *Wojnowicia sp.*

19. Procédé selon la revendication 18, **caractérisé en ce qu'**on effectue l'hydroxylation avec *Absidia orchidis, Absidia coerulea, Acremonium strictum, Ascochyta clematidina, Aspergillus alliaceus, Aspergillus awamori, Aspergillus fischeri, Aspergillus flavus, Aspergillus malignus, Aspergillus melleus, Aspergillus nidulans, Aspergillus niger, Aspergillus ochraceus, Aspergillus variecolor, Bacillus megaterium, Beauveria bassiana, Beauveria tenella, Botryodiplodia malorum, Caldariomyces fumago, Calonectria decora, Colletotrichum phomoides, Curvularia lunata, Fusa rium oxysporum, Fusarium solani, Gibberella zeae, Glomerella cingulata, Gloeosporium fructigenum, Gloeosporium higgensianum, Gloeosporium kaki, Gloeosporium lacticolor, Gloeosporium olivarum, Glomerella fusaroides, Gnomonia cingulata, Haplosporella hesperedica, Helminthosporium sp., Helicostylum piriforme, Metarhizium anisopliae, Mucor plumbeus, Mucor spinosus, Nigrospora sphaerica, Rhizopus arrhizus, Rhizopus cohnii, Rhizopus delemar, Rhizopus japonicus, Rhizopus kazaensis, Rhizopus microsporus, Rhizopus oryzae, Rhizopus shanghaiensis, Rhizopus stolonifer, Rhizopus tritici, Sporotrichum sulfurescens, Syncephalastrum racemosum, Wojnowicia graminis* et *Wojnowicia hirta.*

20. Procédé selon la revendication 19, **caractérisé en ce qu'**on effectue l'hydroxylation avec *Absidia orchidis* (ATCC 6647), *Acremonium strictum* (NRRL 5759), *Ascochyta clematidina* (CBS), *Aspergillus alliaceus* (ATCC 10060), *Aspergillus awamori* (CBS), *Aspergillus fischeri* (ATCC 1020), *Aspergillus malignus* (IMI 16061), *Aspergillus melleus* (CBS), *Aspergillus nidulans* (ATCC 11267), *Aspergillus niger* (ATCC 9142, ATCC 11394), *Aspergillus ochraceus* (NRRL 405, NRRL 410, CBS 13252, ATCC 46504), *Aspergillus variecolor* (ATCC 10067), *Bacillus megaterium* (ATCC 13368), *Beauveria bassiana* (IFO 5838, ATCC 13144, IFO 4848, CBS 11025, CBS 12736, ATCC 7159), *Botryodiplodia malorum* (CBS 13450), *Caldariomyces fumago* (ATCC 16373), *Calonectria decora* (ATCC 14767), *Curvularia lunata* (IX 3, NRRL 2380), *Fusarium solani* (ATCC 12823), *Fusarium oxysporum* (ATCC 7808), *Gibberella* zeae (CBS 4474), *Glomerella cingulata* (ATCC 12097, ATCC 10534, CBS 23849, CBS 23749, ATCC 16646, IFO 6459, IFO 6425, IFO 6470, ATCC 15093, ATCC 10529, IFO 5257, ATCC 56596, ATCC 64682), *Glomerella fusaroides* (ATCC 9552), *Gnomonia cingulata* (CBS 15226), *Haplosporella hesperedica* (CBS 20837), *Helicostylum piriforme* (ATCC 8992), *Helminthosporium sp.* (NRRL 4671), *Metarhizium anisopliae* (IFO 5940), *Mucor plumbeus* (CBS 29563), *Nigrospora sphaerica* (ATCC 12772), *Rhizopus arrhizus* (ATCC 11145), *Rhizopus oryzae* (ATCC 4858, ATCC 34102, CBS 32947), *Rhizopus stolonifer* (ATCC 15441), *Syncephalastrum racemosum* (IFO 4827) et *Wojnowicia graminis* (CBS 89168).
